(19)

**Europäisches Patentamt**
**European Patent Office**
**Office européen des brevets**

(11)  **EP 1 806 091 B1**

(12)  **EUROPEAN PATENT SPECIFICATION**

(45) Date of publication and mention
of the grant of the patent:
**26.11.2014 Bulletin 2014/48**

(51) Int Cl.:
*A61B 1/00* *(2006.01)*          *A61B 1/04* *(2006.01)*
*G06T 1/00* *(2006.01)*          *A61B 5/07* *(2006.01)*
*A61B 1/05* *(2006.01)*

(21) Application number: **05799224.0**

(22) Date of filing: **27.10.2005**

(86) International application number:
**PCT/JP2005/019771**

(87) International publication number:
**WO 2006/046637 (04.05.2006 Gazette 2006/18)**

(54) **IMAGE PROCESSING METHOD**

BILDBEARBEITUNGSVERFAHREN

PROCEDE DE TRAITEMENT D'IMAGES

(84) Designated Contracting States:
**DE FR GB**

(30) Priority: **29.10.2004   JP 2004316968**

(43) Date of publication of application:
**11.07.2007   Bulletin 2007/28**

(73) Proprietor: **Olympus Corporation**
**Tokyo 151-0072 (JP)**

(72) Inventors:
• **NISHIMURA, Hirokazu,**
**c/o Olympus Medical System Corp.**
**Tokyo 151-0072 (JP)**

• **HASEGAWA, Jun c/o Olympus Medical System Corp.**
**Tokio 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**EP-A- 1 437 083          JP-A- 2 131 686**
**JP-A- 2003 079 568     JP-A- 2003 093 328**
**JP-A- 2003 284 682     JP-A- 2004 041 709**
**JP-A- 2004 294 788     US-A- 5 469 840**

**Description**

Technical Field

The present invention relates to an image processing method and a capsule type endoscope device which detects images inappropriate for diagnosis in endoscopic images of a body cavity captured by an endoscope device and controls display and/or storage of the inappropriate images.

Background Art

In the medical field, observation and diagnosis of organs in a body cavity using medical equipment having an image capturing function such as an X-ray, CT, MRT, ultrasonic observing devices, endoscope devices and the like are widely practiced. In the medical equipment having the image capturing function, an endoscope device, for example, captures an image of an organ in a body cavity using image pickup means such as a solid image pickup device or the like by inserting an elongated insertion portion into the body cavity and taking in the image by an objective optical system provided at a tip end portion of the insertion portion. The endoscope device displays the endoscopic image of the organ in the body cavity on a monitor screen on the basis of an image pickup signal so that an operator performs observation and diagnosis from the endoscopic image displayed on the monitor screen.

Since this endoscope device is capable of direct capturing of an image of a digestive tract mucous, the tone of the mucous, lesion shape, micro structure of the mucous surface and the like can be comprehensively observed.

Recently, a capsule type endoscope device has been developed as medical equipment having a new image capturing function for which usability can be expected similar to this endoscope device. In general, the capsule type endoscope device comprises a capsule type endoscope which is swallowed through the mouth of a subject and captures an image of inside of the digestive organ in the course of advance through the digestive tract in the body, a captured image pickup signal being transmitted to the outside the body, a receiver for receiving the transmitted image pickup signal outside the body and recording/accumulating the received image pickup signal, and an observing device for displaying the picked-up image on a monitor on the basis of the image pickup signal recorded/accumulated in this receiver. The capsule type endoscope device thus configured is disclosed in Japanese Unexamined Patent Application Publication No. 2000-23980.

The above capsule type endoscope device keeps on capturing images of the inside of the body cavity from oral swallowing of the capsule type endoscope by the subject to excretion to the outside of the body, and captured image pickup signals are transmitted to a receiver provided outside the body. It takes several hours from when the capsule-type endoscope is swallowed by the subject till when it is excreted to the outside of the body, passing through each digestive tract of an esophagus, a gaster, a duodenum, a small intestine and a colon in the body cavity. For example, suppose that the capsule type endoscope captures two images (frames) per second and transmits them to the receiver outside the body and it takes 6 hours from when the capsule type endoscope is swallowed and till when it is excreted to the outside of the body, the number of captured images amounts to the huge number of 43200 images. If these images are all to be recorded, an extremely large capacity is needed for a recording device, which causes a problem of increased cost of the device. In order to solve this problem, there can be a method for reducing the capacity of the image using an image compression processing, for example. With this method, the quality of the recorded image is deteriorated, which makes observation of a fine structure of the mucous surface and a subtle change in the tone difficult.

Also, if this huge number of images are to be displayed on an observing device for observation and diagnosis, suppose that ten images per second are displayed, for example, it takes a relatively long time of 72 minutes to display all the images. An operator should observe the captured images displayed for this long time, and there is a problem that the time load is excessive for the operator. In order to solve this problem, there can be a method for reducing an observing time by increasing the number of images displayed per unit time. With this method, however, there is a limitation in prevention of oversight of a lesion portion and the possible number of displayed images per unit time in view of realization of accurate observation diagnosis, and reduction of observing time by the operator becomes difficult.

On the other hand, images of an organ in the body cavity captured by the capsule type endoscope device are not necessarily images appropriate for diagnosis. For example, there can be an image in which an extremely dark space or high light occupies the majority of the visual field, an image where the entire visual field is in a tone such as red or yellow found in the case of excessive close-up to or contact with a living mucous (an image commonly called by physicians as "red ball" captured in a state too close to a wall of a digestive tract), and an image showing a residue (foreign substance) such as stool in a colon. These images not appropriate for diagnosis (hereinafter referred to as inappropriate images) are often observed in normal endoscopic observation. In the capsule type endoscope moving and obtaining a visual change basically using a peristaltic motion of the digestive tract similar to food, the similar inappropriate images might appear continuously due to retention.

Document EP 1 437 083 A1 discloses an endoscope inserting direction detecting method, wherein a red image is acquired from among red, green, and blue images constituting a received endoscopic image. Further, a gradient vector is calculated in order to obtain a direction of a gradient in brightness. Pixels constituting the received data of the red image may be binary-coded using a threshold, and halation pixels are sampled. Alternatively, a number of pixels representing a shade can be calculated, wherein it is determined whether the number of the shade pixels is large enough to judge that a lumen is found in a field of view. Finally, it is judged whether a lumen visualized in the received endoscopic image can be found within the field of view or the direction of a lumen can be detected in the received endoscopic image. If the endoscope is approached too closely to the mucosa, information concerning manipulations of the endoscope are produced on the basis of the result of the detection.

Document US 5,469,840 A discloses an electromotive warping type endoscope with velocity control. If an insertion portion of the endoscope is inserted into a colon of a human, a full red image can be displayed on a monitor when the leading portion of the insertion portion comes in contact with a tract wall, whereby a dangerous state is detected. Further, an automatic light adjusting circuit may detect the brightness level of an observed image in response to an output video signal. In response to the brightness level signal, a motor control unit may discriminate that a subject is positioned closely if the brightness level of light reflected by the subject to be inspected is high. Moreover, a dark extraction portion can convert each pixel of the supplied image into binary data. In order to eliminate a small dark region generated due to bending of an image guide, which is made of a fiber bundle and a TV camera, at the time of extracting the dark portion, an average value of data about pixels surrounding a certain pixel may be used as data about the certain pixel.

Document JP 2003 284682 A describes an electronic endoscope device having a histogram circuit which produces histogram data from an image signal. A rate of halation occurrence is calculated based on the histogram data and the presence or absence of halation occurrence is detected.

Then, the present invention has an object to provide an image processing method which can determine if an image obtained by capturing an image of a subject is inappropriate for observation and diagnosis or not and a capsule type endoscope device provided with an image processing device which can determine if an image obtained by capturing an image of a subject is inappropriate for observation and diagnosis or not.

Disclosure of Invention

Means for Solving the Problem

An image processing method according to claim 1 is defined.
Further aspects of the invention are explained in the dependent claim.

Brief Description of the Drawings

Fig. 1A is a block diagram illustrating an outline configuration of a capsule type endoscope device 1 using an image processing method according to the present invention.
Fig. 1B is a block diagram illustrating an outline configuration of a terminal device 7 using the image processing method according to the present invention.
Fig. 2 is an explanatory diagram for explaining an outline structure of a capsule type endoscope 3 of the capsule type endoscope device 1.
Fig. 3 is a block diagram illustrating an outline internal configuration of the capsule type endoscope device 1.
Fig. 4 is an explanatory diagram for explaining a signal configuration transmitted from the capsule type endoscope 3.
Fig. 5 is an explanatory diagram for explaining position detection of the capsule type endoscope 3.
Fig. 6 is an explanatory view for explaining an antenna unit 4 of the capsule type endoscope device 1.
Fig. 7 is an explanatory view for explaining a shield jacket 72 of the capsule type endoscope device 1.
Fig. 8 is an explanatory view for explaining an attached state of an external device 5 of the capsule type endoscope device 1 to a subject.
Fig. 9 is a bock diagram illustrating a configuration of the capsule type endoscope 3.
Fig. 10 is a flowchart for explaining a processing operation relating to determination of a dark space image.
Fig. 11 is a flowchart for explaining a processing operation relating to determination of a high light image.
Fig. 12 is a flowchart for explaining a processing operation relating to determination of a foreign substance image.
Fig. 13 is an explanatory diagram for explaining an array table used for calculation of a parameter representing a tone of a pixel.
Fig. 14 is an explanatory diagram for explaining distribution areas of a living mucous surface pixel and a foreign substance pixel in a two-dimensional area with two parameters representing tones of pixels as axes.
Fig. 15 is a flowchart for explaining a processing operation when a dark space image, a high light image and a foreign substance image are determined in a series of procedures.

Fig. 16 is a flowchart for explaining a processing operation relating to determination of an excessively close-up image.

Fig. 17 is a flowchart for explaining a processing operation relating to determination of other observation inappropriate images.

Fig. 18 is an outline diagram for explaining a frequency characteristic of a digital filter used in the present embodiment.

Fig. 19A is a diagram for explaining fluctuation of a band filtering result at a high light peripheral boundary portion, and Fig. 19A is an explanatory diagram for explaining a position of high light in an image.

Fig. 19B is a profile for explaining a pixel value in a-a' section of Fig. 19A.

Fig. 19C is an explanatory diagram for explaining a result obtained by applying a band filtering to an image of Fig. 19A.

Fig. 19D is a profile for explaining a pixel value in b-b' section of Fig. 19C.

Fig. 20 is a flowchart for explaining a processing operation relating to determination of an inappropriate image.

Fig. 21 is a flowchart for explaining an image display operation in the terminal device 7.

Fig. 22 is a flowchart for explaining an image storage operation in the terminal device 7.

Best Mode for Carrying Out the Invention

Embodiments of the present invention will be described below referring to the attached drawings.

(First embodiment)

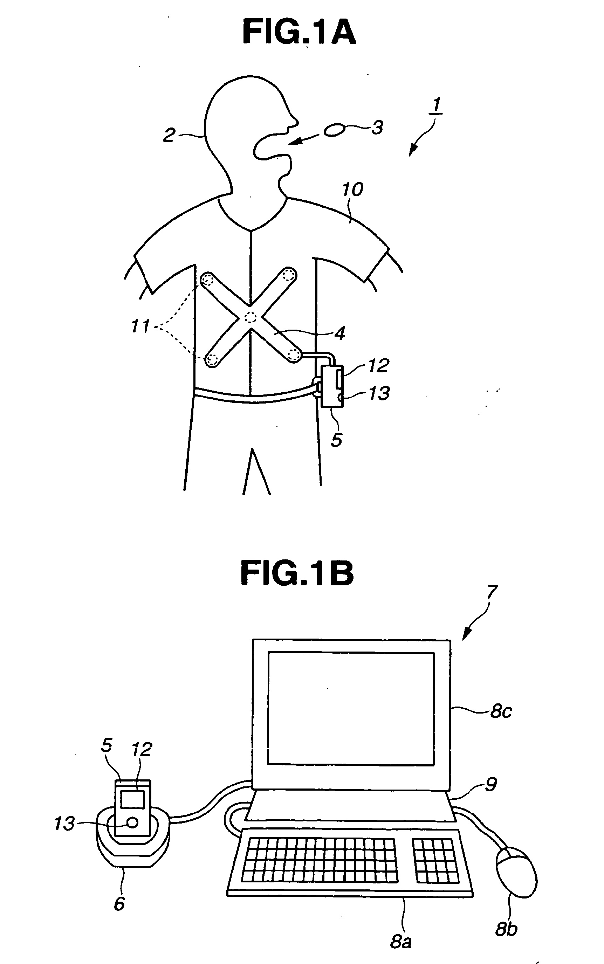

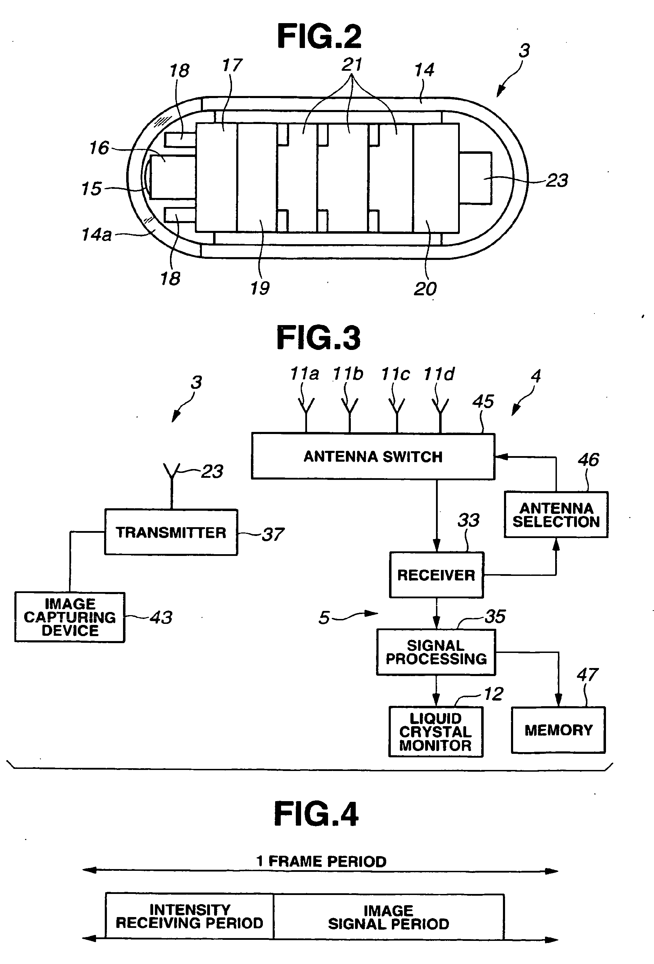

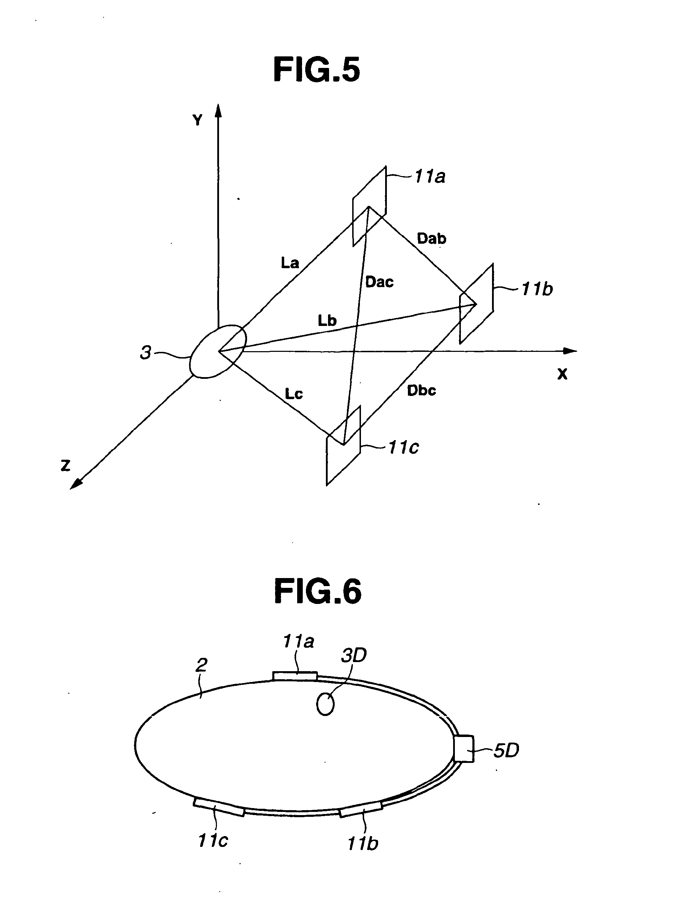

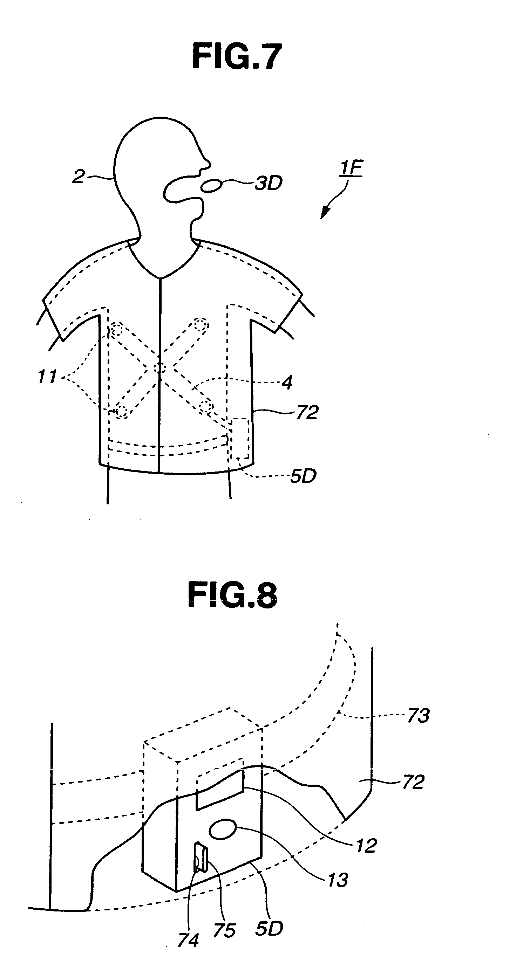

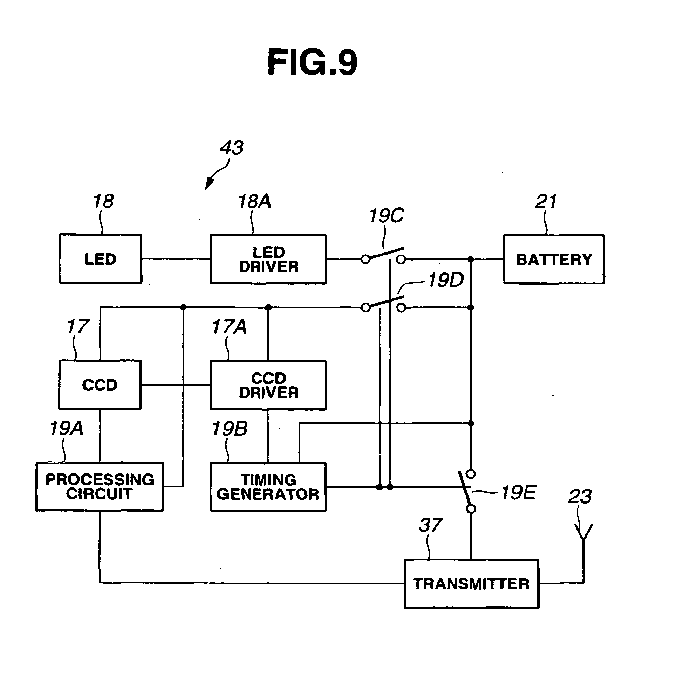

First, a capsule type endoscope device and an image processing method according to a first embodiment of the present invention will be described using the attached drawings. First, the capsule type endoscope device according to the first embodiment of the present invention will be described using Figs. 1 to 9. Fig. 1A is a block diagram illustrating an outline configuration of the capsule type endoscope device 1 using the image processing method according to the present invention. Fig. 1B is a block diagram illustrating an outline configuration of a terminal device 7 using the image processing method according to the present invention. Fig. 2 is an explanatory diagram for explaining an outline structure of a capsule type endoscope 3 of the capsule type endoscope device 1. Fig. 3 is a block diagram illustrating an outline internal configuration of the capsule type endoscope device 1. Fig. 4 is an explanatory diagram for explaining a signal configuration transmitted from the capsule type endoscope 3. Fig. 5 is an explanatory diagram for explaining position detection of the capsule type endoscope 3. Fig. 6 is an explanatory view for explaining an antenna unit 4 of the capsule type endoscope device 1. Fig. 7 is an explanatory view for explaining a shield jacket 72 of the capsule type endoscope device 1. Fig. 8 is an explanatory view for explaining an attached state of an external device 5 of the capsule type endoscope device 1 to a subject. Fig. 9 is a block diagram illustrating a configuration of the capsule type endoscope 3.

The capsule endoscope device 1 as an image capturing device using the image processing method of the present invention comprises, as shown in Fig. 1A, the capsule type endoscope 3, the antenna unit 4, and the external device 5. The capsule type endoscope 3 is formed in a shape that is swallowed from the mouth of a patient, who is a subject, into the body cavity and advances through a digestive tract by a peristaltic motion, though the detail will be described later. Also, the capsule type endoscope 3 has inside an image capturing function for capturing an image inside of the digestive tract and generating its captured image information and a transmission function for transmitting the captured image information to outside the body. The antenna unit 4 is provided on the body surface of the patient 2, though its detail will be described later. Also, the antenna unit 4 has a plurality of antennas 11 for receiving the captured image information transmitted from the capsule type endoscope 3. The external device 5 has its outer shape formed in a box state and has functions for various processing of the captured image information received by the antenna unit 4, recording of the captured image information, display of captured images by means of the captured image information and the like, though the details will be described later. On the surface of the exterior of this external device 5, a liquid crystal monitor 12 for displaying the captured image and an operation portion 13 for giving operation instructions of the various functions are provided.

At this external device 5, an LED for displaying an alarm on a remaining amount of a battery for a driving power supply and a power switch as the operation portion 13 are provided. Also, a calculation execution portion using a CPU and a memory may be provided inside the capsule type endoscope 3 so that the image processing method according to the present invention is executed for the received and recorded captured image information, which will be described later.

This external device 5 is attached to the body of the patient 2, and as shown in Fig. 1B, it is connected to a terminal device 7 as an image processing device by being attached to a cradle 6. A personal computer, for example, is used as the terminal device 7, and it comprises a terminal body 9 having a processing function and a storage device (storing function) of various data, a keyboard 8a and a mouse 8b for input of various operation processing, and a display 8c as a display device for displaying various processing results. A basic function of the terminal device 7 is to take in the captured image information stored in the external device 5 through the cradle 6, to write and record it in a rewritable memory built in the terminal body 9 or a portable memory such as a rewritable semiconductor memory or the like which can be detachably attached to the terminal body 9, and to execute image processing for displaying the recorded captured image information on the display 8c. The captured image information stored in the external device 5 may be taken in the terminal device 7 through an USB cable or the like instead of the cradle 6. The cradle 6 or the like is image input means for inputting an image captured by the capsule type endoscope 3.

In the image processing of the terminal device 7, selection of an image to be displayed from the captured image information taken in and recorded from the external device 5 according to an elapsed time, detection of an image inappropriate for diagnosis by the image processing method according to an embodiment, which will be described later, and the like are executed.

Next, the outer shape and internal structure of the capsule type endoscope 3 will be described using Fig. 2. The capsule type endoscope 3 is formed into a capsule shape made of an exterior member 14 with the U-shaped section and a cover member 14a substantially in the semi-spherical shape formed of a transparent member attached to an open end at the tip end side of the exterior member 14 in a water-tight manner.

In an internal hollow portion of the capsule shape made of the exterior member 14 and the cover member 14a, inside an arc-state center portion of the semi-sphere of the cover member 14a, an objective lens 15 for taking in an image of an observed portion incident through the cover member 14a is stored and arranged at a lens frame 16. At an image forming position of this objective lens 15, a charge coupled device, which is an image capturing device (hereinafter referred to as CCD) 17 is arranged. Around the lens frame 16 for storing the objective lens 15, four white LED 18 for emitting illumination light are arranged on the same plane (only two LED of them are shown in the figure). In a hollow portion of the exterior member 14 at the rear end side of the CCD 17, a processing circuit 19 for generation of an image pickup signal photoelectrically converted by driving control of the CCD 17, image capturing processing for generating a captured image signal by applying predetermined signal processing to the image pickup signal, and processing of LED driving for controlling turning on/off operation of the LED 18, a communication processing circuit 20 for converting the captured image signal generated by the image capturing processing of the processing circuit 19 to a wireless signal and transmitting it, a transmission antenna 23 for transmitting a wireless signal from the communication processing circuit 20 to the outside, and a plurality of button-type batteries 21 for supplying driving power to the processing circuit 19 and the communication processing circuit 20.

The CCD 17, the LED 18, the processing circuit 19, the communication processing circuit 20, and the transmission antenna 23 are arranged on boards, not shown. The boards are connected by a flexible board. The processing circuit 19 is provided with a calculation circuit, not shown, for image processing, which will be described later. That is, the capsule type endoscope 3 comprises, as shown in Fig. 3, an image capturing device 43 made of the CCD 17, the LED 17, and the processing circuit 19, a transmitter 37 including the communication processing circuit 20, and the transmission antenna 23.

Next, detailed configuration of the image capturing device 43 of the capsule type endoscope 3 will be described using Fig. 9. The image capturing device 43 comprises an LED driver 18A for controlling operation of turning on/off of the LED 18, a CCD driver 17A for transferring a charge photoelectrically converted by control of the driving of the CCD 17, a processing circuit 19A for generating an image pickup signal using the charge transferred from the CCD 17 and generating an captured image signal by applying predetermined signal processing to the image pickup signal, a switch 19 for supplying driving power from the battery 21 to the LED driver 18A, the CCD driver 17A, the processing circuit 19A and the transmitter 37, and a timing generator 19B for supplying a timing signal to the switch 19 and the CCD driver 17A. The switch 19 comprises a switch 19C for turning on/off power supply from the battery 21 to the LED driver 18A, a switch 19D for turning on/off power supply to the CCD 17, the CCD driver 17A, and the processing circuit 19A, and a switch 19E for turning on/off power supply to the transmitter 37. To the timing generator 19B, driving power is supplied from the battery 21 all the time.

The image capturing device 43 of the capsule type endoscope 3 in this configuration is in the non-operated state except the timing generator 19B when the switches 19C to 19E are in the off state. When the switch 19D is turned on by a timing signal from the timing generator 19B, power is supplied to the CCD 17, the CCD driver 17A, and the processing circuit 19A to bring them into the operated state.

After an unnecessary dark current is eliminated by operating an electronic shutter of the CCD 17 at the beginning of driving of the CCD 17, the timing generator 19B turns on the switch 19C so as to drive the LED driver 18A to turn on the LED 18 and expose the CCD 17. The LED 18 lights the CCD 17 for a predetermined exposure time and then, turns off the switch 19C so as to reduce power consumption, and the LED 18 is turned off.

A charge accumulated during the exposure time of the CCD 17 is transferred to the processing circuit 19A by means of control of the CCD driver 17A. At the processing circuit 19A, an image pickup signal is generated based on the charge transferred from the CCD 17, and predetermined signal processing is applied to the image pickup signal so as to generate an endoscopic image signal. The CCD 17, the CCD 17A, and the processing circuit 19A constitute image generating means. With regard to the endoscopic image signal, if a signal transmitted from the transmitter 37 is an analog wireless type, for example, an analog captured image signal in which a complex synchronous signal is superimposed on a CDS output signal is generated and outputted to the transmitter 37. If it is a digital wireless type, the captured image signal is converted to a digital signal by an analog/digital converter and then, converted to a serial signal and given encoding processing such as scramble, and a digital captured image signal is outputted to the transmitter 37.

The transmitter 37 applies modulation processing to an analog or a digital captured image signal supplied from the processing circuit 19A and transmits it to the outside from the transmission antenna 23 in a wireless manner. At this time, the switch 19E is operated on/off so that driving power is supplied to the transmitter 37 only when a captured image signal is outputted from the processing circuit 19A by the timing generator 19B.

The switch 19E may be controlled so that it supplies the driving power to the transmitter 37 after a predetermined time has elapsed since the captured image signal is outputted from the processing circuit 19A. Also, it may be so constructed that a pH value of a predetermined value is detected by a pH sensor provided in the capsule type endoscope 3, not shown, or a humidity above a predetermined value is detected by a humidity sensor provided in the capsule type endoscope 3. Alternately, insertion into the body cavity of the patient 2, who is a subject, may be detected by detection of a pressure or acceleration above a predetermined value by a pressure sensor or an acceleration sensor, not shown, so that the switch 19E is controlled to supply the power to the transmitter 37 on the basis of this detection signal.

The image capturing device 43 of the capsule type endoscope 3 usually captures two images per second (2 frames per second = 2 fps) but in the case of an inspection of an esophagus, 15 to 30 images per second (15 to 30 fps) shall be able to be captured. Specifically, a timer circuit, not shown, is provided in the capsule type endoscope 3, and within a predetermined time of a timer count by this timer circuit, images shall be captured at a high speed with more captured images per second. After the predetermined time has elapsed, driving of the image capturing device 43 is controlled so that the image capturing shall be made at a low speed with fewer captured images per second. Alternately, the timer circuit may be operated at the same time as power on of the capsule type endoscope 3 so that the high-speed image capturing is controlled to be carried out till when the endoscope has passed through the esophagus immediately after swallowing by the patient 2. Moreover, a capsule type endoscope for low-speed image capturing and a capsule type endoscope for high-speed image capturing may be separately provided so that they can be used separately according to an observation target portion.

Next, the antenna unit 4 provided on the body surface of the patients 2 will be described. As shown in Fig. 1A, in the case of an endoscopic inspection by swallowing the capsule type endoscope 3, the patient 2 wears a jacket 10 on which the antenna unit 4 comprised by a plurality of receiving antennas 11 is installed. This antenna unit 4 is arranged, as shown in Fig. 6, so that the plurality of receiving antennas 11 having a single directionality such as a patch antenna used in GPS are directed to the intra-body direction of the patient 2. That is, since a capsule body 3D of the capsule type endoscope 3 is retained in the body, the plurality of antennas 11 are arranged so as to surround the capsule body 3D in the body. By using the antennas 11 with high directionality, an influence of interference disturbance by an electric wave from appliances or the like other than the capsule body 3D in the body hardly occurs.

The jacket 10 is a shield jacket 72 formed by an electromagnetic shield fiber so as to cover the antenna unit 4 installed on the body surface of the patient 2 and a body portion 5D of the external device 5 installed at the hip of the patient 2 by a belt. For the electromagnetic fiber forming this shield jacket 72, a metal fiber, a metal chemical fiber, copper sulfide containing fiber and the like are used. This shield jacket 72 may be in a vest or a one-piece shape other than the jacket shape.

Also, as an example to attach the external device 5 to the shield jacket 72, as shown in Fig. 8, a key hole 74 is provided at the external body 5D of the external device 5, and a key 75 provided at the shield jacket 72 is inserted into the key hole 74 so that it can be detachably attached to a belt 73. Alternately, a pocket, not shown, may be simply provided at the shield jacket 72 so that the external body 5D is stored. Alternately, a Velcro (registered trademark) may be provided at the external body 5D of the external device 5 and the shield jacket 72, and the Velcro may be used for mounting and fixing.

That is, by wearing the shield jacket 72 on a body on which the antenna unit 4 is arranged, an electric wave to the antenna unit 4 from the outside is shielded, and an influence of interference disturbance by the external electric wave becomes harder to occur.

Next, configuration of the antenna unit 4 and the external device 5 will be described using Fig. 3. The antenna unit 4 comprises a plurality of receiving antennas 11a to 11d for receiving a wireless signal transmitted from the transmission antenna 23 of the capsule type endoscope 3 and an antenna switch 45 for switching the antennas 11a to 11d. The external device 5 comprises a receiving circuit 33 for carrying out receiving processing such as conversion of a wireless signal from the antenna switch 45 to a captured image signal, amplification and the like, a signal processing circuit for generating a signal for displaying a captured image and captured image data by applying predetermined signal processing to the captured image signal supplied from the receiving circuit 33, a liquid crystal monitor 12 as a display device for displaying the captured image by the signal for displaying captured image generated by the signal processing circuit 35, a memory 47 as a storage device for storing captured image data generated by the signal processing circuit 35, and an antenna selection circuit 46 for controlling the antenna switch 45 according to the size of the wireless signal given receiving processing by the receiving circuit 33.

The plurality of receiving antennas 11 shown as the receiving antennas 11a to 11d of the antenna unit 4 in the figure receive a wireless signal transmitted from the transmission antenna 23 of the capsule type endoscope 3 at a predetermined wave intensity. With regard to the plurality of receiving antennas 11a to 11d, the antenna switch 45 is controlled by an antenna selection signal from the antenna selection circuit 46 of the external device 5 so that the receiving antenna to receive the wireless signal is switched sequentially. That is, the wireless signal received by the receiving antennas 11 a to 11 d sequentially switched by the antenna switch 45 is outputted to the receiving circuit 33. At the receiving circuit 33, the receiving intensity of the wireless signal of each of the receiving antennas 11a to 11d is detected, the positional relation of each of the receiving antennas 11a to 11d and the capsule type endoscope 3 is calculated, and the wireless signal is demodulated and a captured image signal is outputted to the signal processing circuit 35. The antenna selection circuit 46 is controlled by output from the receiving circuit 33.

Operation of the antenna switch 45 by the antenna selection circuit 46 will be described. The wireless signal transmitted from the capsule type endoscope 3 is transmitted with an intensity receiving period, which is a transmission period of a receiving intensity signal indicating the receiving intensity of the captured image signal and a video signal period, which is a transmission period of the captured image signal, repeated sequentially, in a transmission period of one frame of a captured image signal, as shown in Fig. 4.

To the antenna selection circuit 46, the receiving intensity of the receiving intensity signal received by each of the receiving antennas 11a to 11d is supplied through the receiving circuit 33. The antenna receiving circuit 46 compares the intensity of the receiving intensity signal of each of the antennas 11a to 11d supplied from the receiving circuit 33. And the antenna selection circuit determines the best antenna to receive the captured image signal of the video signal period, that is, an antenna 11i (i = a to d) with the receiving intensity signal with the highest intensity, and generates and outputs a control signal for switching the antenna switching circuit 45 to that antenna 11i. By this, when the receiving intensity of the receiving intensity signal of another antenna is higher, the receiving antenna of the video signal period is switched at the subsequent frame.

Every time a wireless signal is received from the capsule type endoscope 3, the receiving intensity of the captured image signal or the receiving intensity signal is compared, and the antenna 11i found by the antenna selection circuit 46, which has received the comparison result, to have the highest receiving intensity is designated as an antenna for receiving an image signal. By this, even if the capsule type endoscope 3 is moved within the body of the patient 2, an image signal obtained by the antenna 11 which can detect a signal with the highest receiving intensity at the moved position can be received. Also, since the moving velocity of the capsule type endoscope 3 in the body is divided into an extremely slow portion and a rapid portion, the antenna switching operation is not necessarily carried out once for one image capturing operation but the antenna switching operation may be carried out once for a plurality of times of image capturing operations in a high-speed image capturing mode or the like.

Since the capsule type endoscope 3 is moving in the body of the patient 2, it may be so constructed that a detection result signal as a result of detection of the wave intensity is sent from the external device 5 with an appropriate time interval and an output at transmission by the capsule type endoscope 3 is updated on the basis of the signal. In this way, even if the capsule type endoscope 3 is moved within the body of the patient 2, an appropriate transmission output can be set, wasteful consumption of energy of the battery 21 can be prevented, and the signal transmission/receiving state can be maintained appropriate.

Next, a method for obtaining information indicating a positional relation among the plurality of receiving antennas 11 and the capsule type endoscope 3 will be described using Fig. 5. In Fig. 5, a case will be explained where the capsule type endoscope 3 is set at an origin of three-dimensional coordinates X, Y, Z, as an example. Also, in order to simplify explanation, three receiving antennas 11a, 11b, 11c will be used for the description below in the plurality of receiving antennas 11a to 11d. Also, in the description below, a distance between the receiving antenna 11a and the receiving antenna 11b is set as Dab, the distance between the receiving antenna 11b and the receiving antenna 11c as Dbc, and the distance between the receiving antenna 11a and the receiving antenna 11 c as Dac. Moreover, a distance between the receiving antennas 11a to 11c and the capsule type endoscope 3 shall have a predetermined distance relation.

As for the wireless signal with a given transmission intensity transmitted by the capsule type endoscope 3, the receiving intensity when received by each of the receiving antenna 11j (j = a, b, c) is a function of a distance $Li$ (i = a, b, c) from the capsule type endoscope 3 (the transmission antenna 23 of the capsule type endoscope 3). Specifically, it depends on the distance $Li$ involving an electric wave attenuation amount. Therefore, the distance $Li$ between the capsule type endoscope 3 and each of the receiving antennas 11j is calculated from the receiving intensity received from the receiving antenna 11j of the wireless signal transmitted from the capsule type endoscope 3. For calculation of the distance $Li$, related data such as an electric wave attenuation amount by the distance between the capsule type endoscope 3 and the receiving antenna 11j is set at the antenna selection circuit 46 in advance. Also, the calculated distance data indicating the positional relation between the capsule type endoscope 3 and each of the receiving antennas 11j is stored in the memory 47 as position information of the capsule type endoscope 3. The captured image information and position information of the capsule type endoscope 3 stored in the memory 47 is useful in position setting of finding of an endoscopic observation in the image processing method by the terminal device 7, which will be described later.

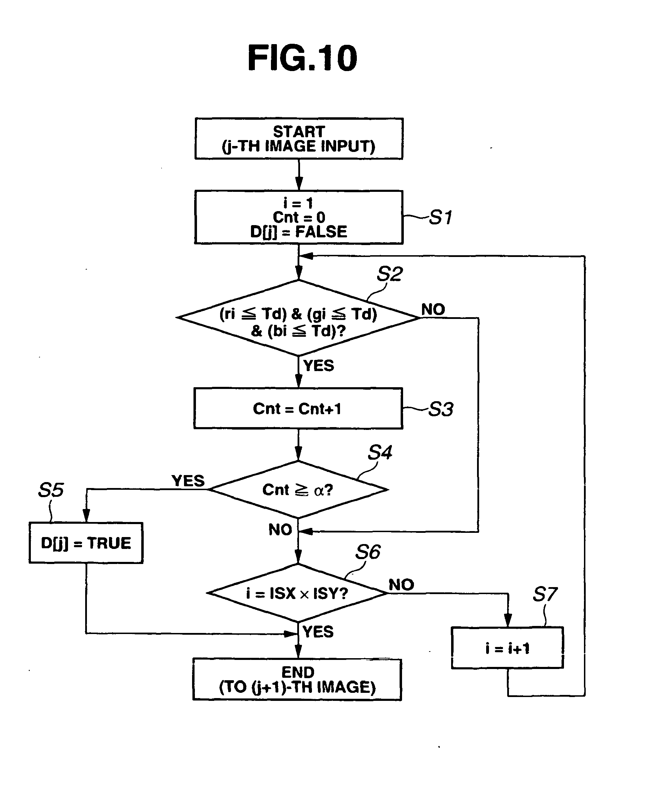

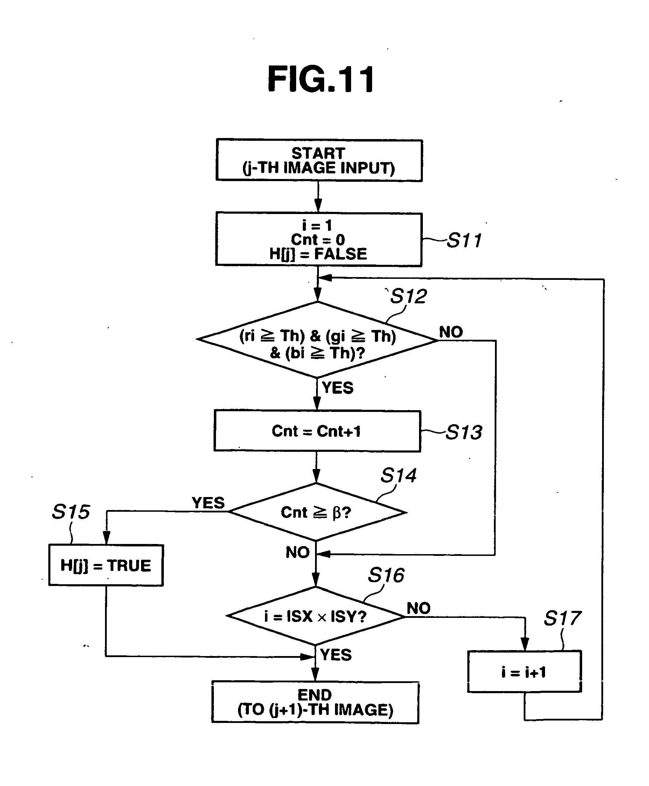

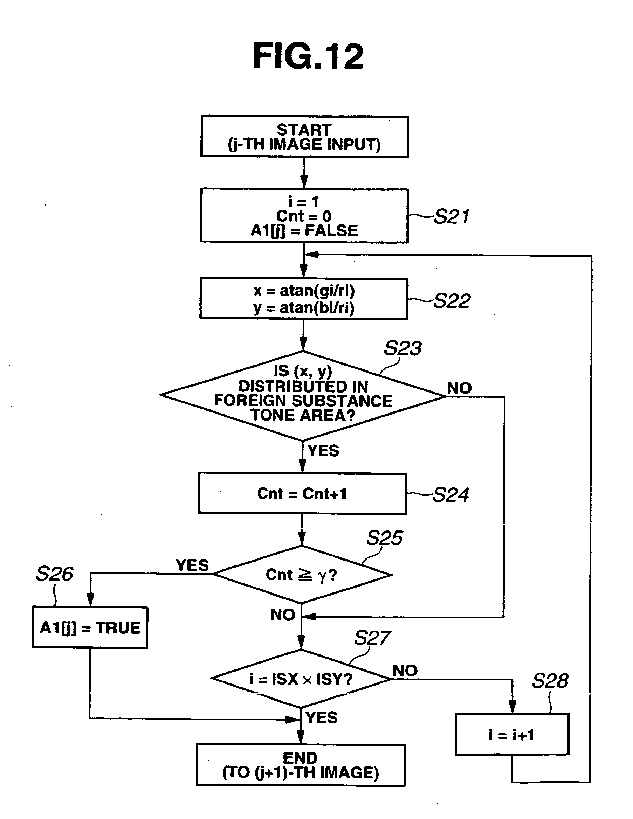

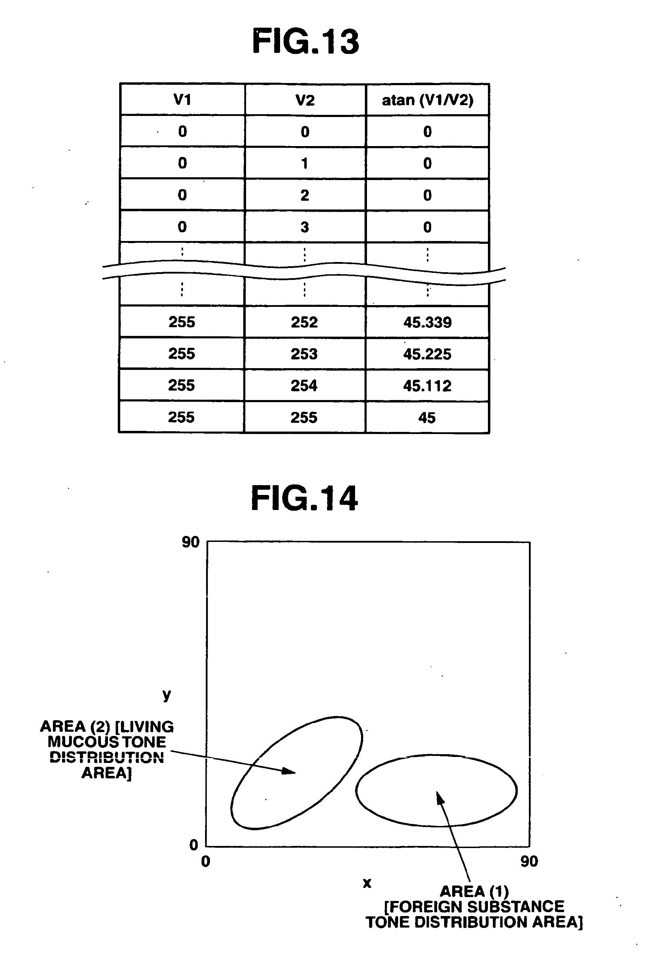

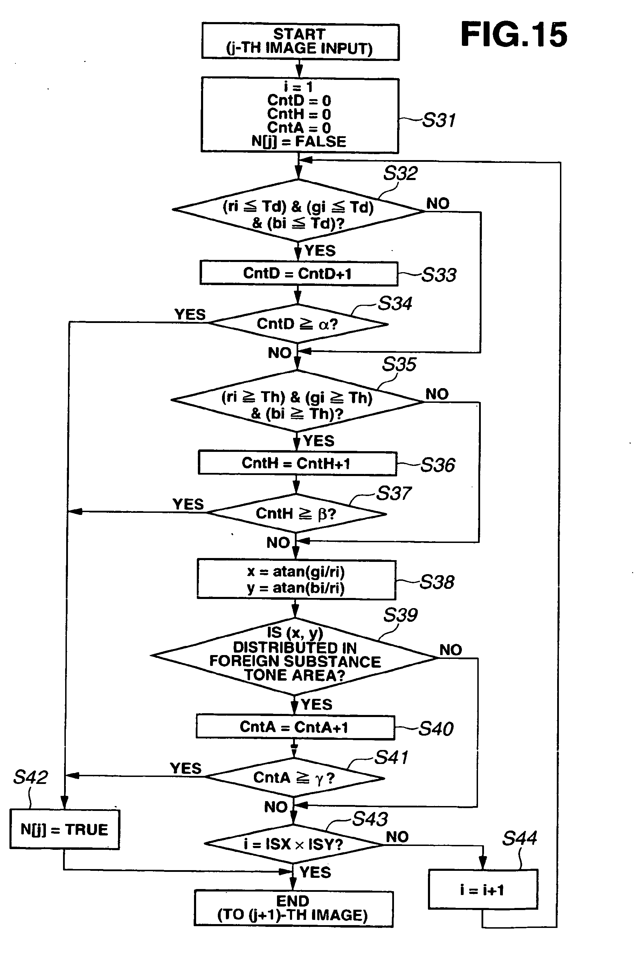

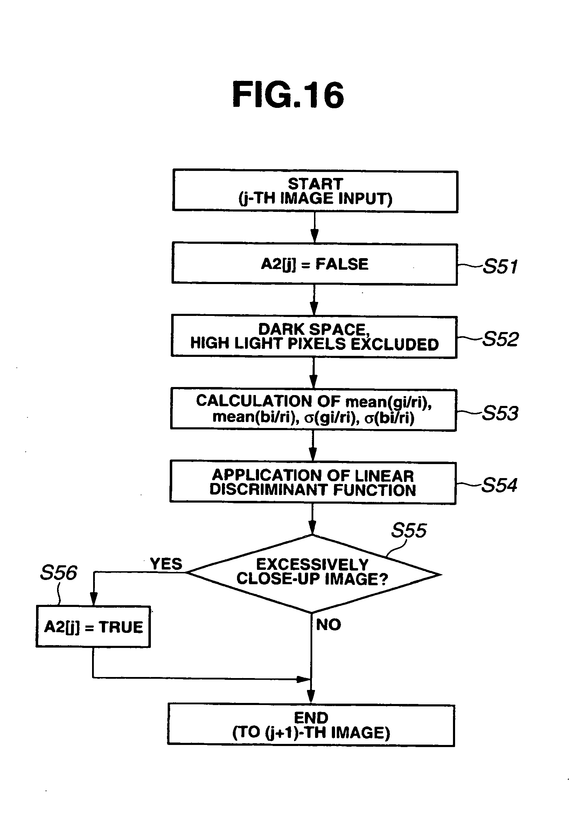

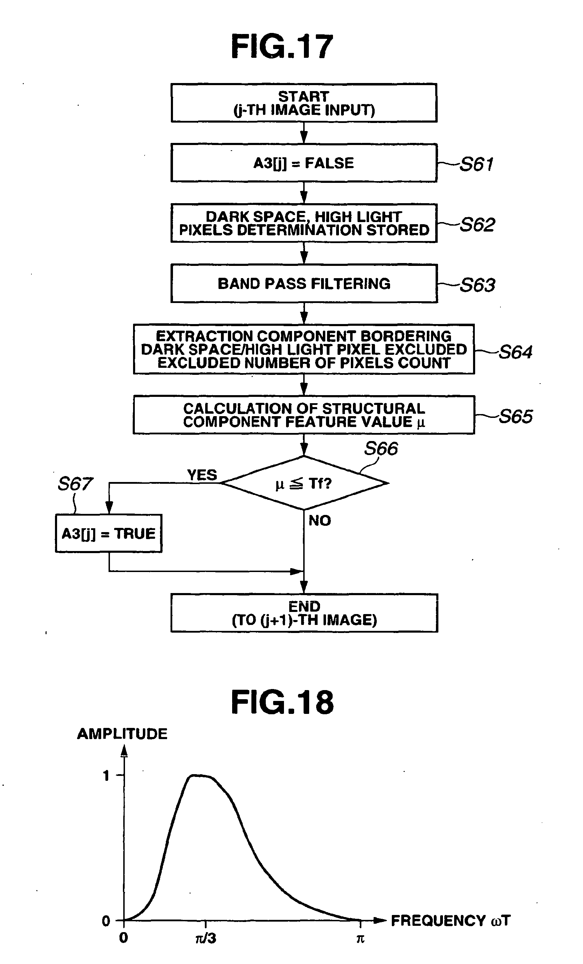

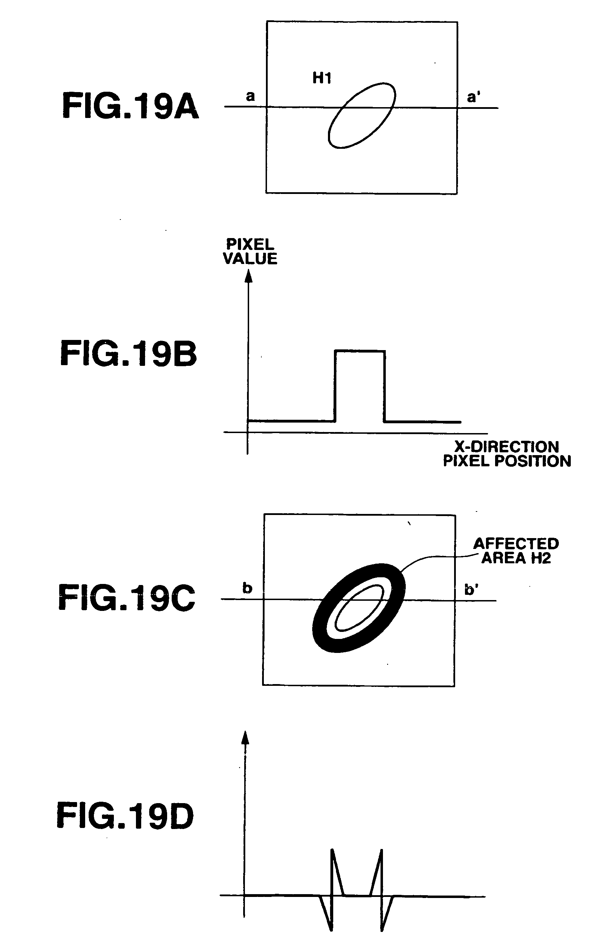

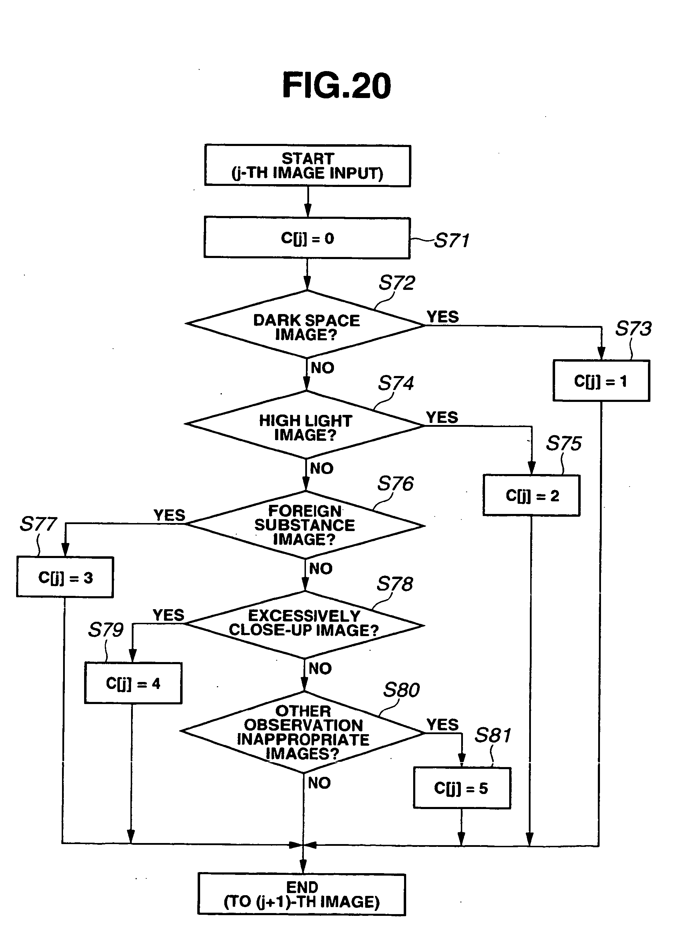

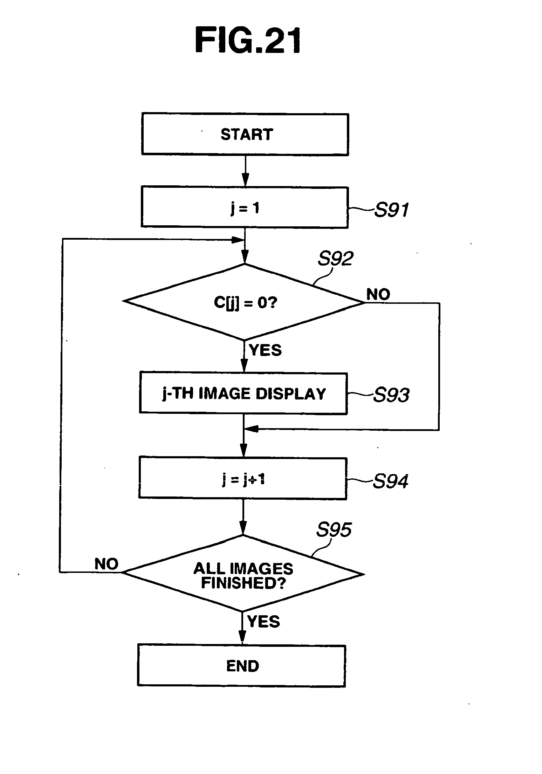

Next, action of the capsule type endoscope device 1 and the image processing method according to the first embodiment of the present invention will be described using Figs. 10 to 21. Fig. 10 is a flowchart for explaining a processing operation relating to determination of a dark space image. Fig. 11 is a flowchart for explaining a processing operation relating to determination of a high light image. Fig. 12 is a flowchart for explaining a processing operation relating to determination of a foreign substance image. Fig. 13 is an explanatory diagram for explaining an array table used for calculation of a parameter representing a tone of a pixel. Fig. 14 is an explanatory diagram for explaining distribution areas of a living mucous surface pixel and a foreign substance pixel in a two-dimensional area with two parameters representing tones of pixels as axes. Fig. 15 is a flowchart for explaining a processing operation when a dark space image, a high light image and a foreign substance image are determined in a series of procedures. Fig. 16 is a flowchart for explaining a processing operation relating to determination of an excessively close-up image. Fig. 17 is a flowchart for explaining a processing operation relating to determination of other observation inappropriate images. Fig. 18 is an outline diagram for explaining a frequency characteristic of a digital filter used in the present embodiment. Fig. 19 is a diagram for explaining fluctuation of a band filtering result at a high light peripheral boundary portion, and Fig. 19A is an explanatory diagram for explaining a position of high light in an image. Fig. 19B is a profile for explaining a pixel value in a-a' section of Fig. 19A. Fig. 19C is an explanatory diagram for explaining a result obtained by applying a band filtering to an image of Fig. 19A. Fig. 19D is a profile for explaining a pixel value in b-b' section of Fig. 19C. Fig. 20 is a flowchart for explaining a processing operation relating to determination of an inappropriate image. Fig. 21 is a flowchart for explaining an image display operation in the terminal device 7.

The image processing method according to the present embodiment is to detect an image inappropriate for observation and diagnosis from a series of images obtained from the capsule type endoscope 3. Also, the capsule type endoscope device 1 according to the present embodiment is operated so that an image recognized as inappropriate upon application of the image processing method is not outputted for display or the like to the terminal device 7 as output means. By preventing output for display or the like of the image determined as inappropriate to the terminal device 7, reduction of time required for observation is enabled. These inappropriate images are inappropriate not only for observation by display on the terminal device 7 but also inappropriate as a target image to be given various image processing. Thus, the image processing method of the present embodiment may be used for eliminating inappropriate images from targets for image processing.

The image processing method to be described is realized by software, and the image processing method can be used in any of the capsule type endoscope 3, the external device 5 or the terminal device 7. Here, an example of application to the terminal device 7 using a personal computer will be explained. Also, in the description of the image processing method, the size of an image is made of three planes of red (R), green (G) and blue (B) of ISX x ISY (1 ≤ ISX, ISY. ISX = 640, ISY = 480, for example), and the number of tones of a pixel in each plane takes 8 bits, that is, a value of 0 to 255.

In the capsule type endoscope 3, when an image is captured so as to contain a living mucous surface or an image pickup target other than the living mucous surface, inappropriate images are captured together with images usable for observation or diagnosis. In these inappropriate images, information of living mucous surface runs short or does not exist in a visual field. Thus, these inappropriate images are images which do not deserve to be stored or observed but often observed in usual endoscopic inspections. The inappropriate images are roughly classified into the following five categories.

The first category is a dark space image. The dark space image is dark in the entire or the majority of the image due to lack of illumination light amount or the like, in which observation of a living mucous surface is difficult or brightness runs short. The second category is a high light image. The high light image is an image in which high light caused when illumination and a living mucous are opposed and brought too close to each other or a mucous membrane or foam liquid or the like exist occupies the majority of the image or which has much high light. The third category is a foreign substance image. The foreign substance image is an image in which residues (foreign substance) such as feces as an image pickup target other than the living mucous surface in a colon occupy the majority of the image due to defective pre-treatment of the endoscopic observation, deterioration of peristaltic motion caused by aging or the like, or an image in which there are many foreign substances in the visual field. Also, as the image pickup targets other than the living mucous surface include moisture or liquid in a digestive tract such as bubbles by digestive juice or the like. The fourth category is an excessively close-up image. The excessively close-up image is an image in which the entire visual field turns red, yellow or the like found in the case of getting too close to or contact with a living mucous (an image commonly called by physicians as "red ball"). Since the excessively close-up image is defocused by excessive close-up and its visual field range is small, discovery of lesions or observation findings of vessel images or the like can not be obtained. The fifth category is other images inappropriate for observation. The other observation inappropriate images include submerged images captured in a state where the capsule type endoscope is submerged under water accumulated in a digestive tract and a visual field flowing image captured in a state where the capsule type endoscope is moved at a high speed or instantaneously, for example, or a radical peristaltic motion occurs due to pulsation or any other reasons. Most of these observation inappropriate images are defocused, and observation of vessel images or a structure of living mucous surface is difficult. The images inappropriate for observation, that is, the image captured in poor state for observation sometimes prevents improvement of efficiency in observation and diagnosis.

For the dark space images, high light images and foreign substance images, pixels and areas of the dark space, high light and foreign substances are detected, respectively, and determination on observation inappropriate image is made on the basis of the number of pixels, a proportion in the total number of pixels in the image or the positions of those pixels. For the excessively close-up images and other observation inappropriate images, a structural component amount of living mucous surface such as tone and profile of the entire image is calculated and determination on observation inappropriate image is made on the basis of this value. The specific image processing method for detecting these inappropriate images will be described below for each category of the inappropriate images. A value of a color signal of the i-th pixel in each image of an R image, a G image and a B image is noted as ri, gi, and bi. Also, known inverse γ correction is supposed to be applied to each image as pre-treatment.

First, the processing operation for determination of the dark space image will be described using Fig. 10. The determination of the dark space image here is made on the basis of the proportion of the pixels of the dark space in the total number of pixels in the image. The captured image signal captured by the capsule type endoscope 3 and transmitted by the external device 5 is given predetermined signal processing at the external device 5 and stored as captured image signal data. The captured image signal data stored in the external device 5 is transferred to/stored in the terminal device 7. The terminal device 7 carries out determination operation of the dark space image on the basis of the stored captured image signal data.

First, at Step S1, i is initialized to 1, which indicates the number specifying a pixel in the captured image signal data of the j-th image (j is an integer equal to or larger than 1) to be processed. Also, a counter Cnt for counting the number of pixels determined as the dark space is initialized to 0. Moreover, a flag D[j] indicating the determination result on whether the j-th image is a dark space image or not is initialized to FALSE. The number i specifying the pixel and the counter Cnt take integers not less than 1 and not more than ISX x ISY, and either one of TRUE indicating determination as a dark space image or FALSE indicating determination as not a dark space image is set to a value of the flag D[j].

Next, at Step S2, it is determined if the i-th pixel belongs to a dark space or not. Specifically, the value of the i-th pixel in each of the R image, the G image and the B image is determined as a pixel belonging to the dark space, if ri ≤ Td, gi ≤ Td and bi ≤ Td for ri, gi and bi. Td is a threshold value of each color, and in the present embodiment of the present invention, it is set as Td = 50, for example. Td is the same value for the R image, the G image and the B image, but since a living mucous has a tendency that the R image is the brightest, the threshold for ri may be set higher than the threshold values for gi and bi. Also, different thresholds may be set for each of ri, gi and bi. If the i-th pixel is determined as a pixel belonging to the dark space, the program goes on to Step S3, while if the i-th pixel is determined as a pixel not belonging to the dark space, the program goes on to Step S6. Steps S2 and S3 constitute a feature value calculation process or feature value calculating means for calculating a feature value on the basis of the value of a pixel, that is, brightness of the image.

At Step S3, the value of Cnt is incremented by 1. Then, at Step S4, it is determined if the j-th image is a dark space image or not. Specifically, it is determined as the dark space image, if Cnt ≥ α. α is a threshold value for specifying how many pixels should exist for the total number of pixels to determine it as the dark space image, that is, a threshold value of an image-captured state to determine if the image is satisfactory or not. In the present embodiment, α is set as α = 0.7 x ISX x ISY, for example, that is, to the number of pixels of 70% in the total number of pixels. In the case of Cnt ≥ α, the program goes on to Step S5, while in the case of Cnt < α, the program goes on to Step S6.

At Step S5, the j-th image to be processed is determined as the dark space image, D[j] = TRUE is set to finish the processing, and the program goes to determination processing for the subsequent (j + 1)-th image. Steps S4 and S5 constitute an image-captured state determining process or image-captured state determining means.

At Step S6, it is determined if the dark space pixel determination at Step S2 has been carried out for all the pixels or not. Specifically, in the case of i < ISX x ISY, 1 is added to the number i specifying the pixel (i = i + 1) at Step S7, Step S2 to Step S6 are executed for the next pixel, and the dark space pixel determination is carried out for the remaining pixels. In the case of i = ISX x ISY, the processing is finished, and the program goes to the determination processing for the subsequent (j + 1)-th image.

As mentioned above, by a series of processing in Steps S1 to S7, determination can be made if the image to be processed is a dark space image or not on the basis of the pixel value of each pixel of the captured image.

Next, the processing operation for determination of a high light image will be described using Fig. 11. The determination of the high light image is made on the basis of a proportion of the high light pixels in the total number of pixels in the image.

First, at Step S11, i is initialized to 1, which indicates the number specifying a pixel in the captured image signal data of the j-th image (j is an integer equal to or larger than 1) to be processed. Also, a counter Cnt for counting the number of pixels determined as the dark space is initialized to 0. Moreover, a flag H[j] indicating the determination result on whether the j-th image is a high light image or not is initialized to FALSE. The number i specifying the pixel and the counter Cnt take integers not less than 1 and not more than ISX x ISY. And either one of TRUE indicating determination as a high light image or FALSE indicating determination as not a high light image is set to a value of the flag H[j].

Next, at Step S12, it is determined if the i-th pixel belongs to an extremely bright pixel, that is, a high light pixel or not. Specifically, the value of the i-th pixel in each of the R image, the G image and the B image is determined as a high light pixel, if ri ≥ Th, gi ≥ Th and bi ≥ Th for ri, gi and bi. Th is a threshold value of each color, and in the present embodiment of the present invention, it is set as Th = 240, for example. Th is the same value for the R image, the G image and the B image, but since a living mucous has a tendency that the R image is the brightest, the threshold for ri may be set higher than the threshold values for gi and bi. Also, different thresholds may be set for each of ri, gi and bi. If the i-th pixel is determined as a high light pixel, the program goes on to Step S 13, while if the i-th pixel is determined as not a high light pixel, the program goes on to Step S16.

At Step S 13, the value of Cnt is incremented by 1. Then, at Step S 14, it is determined if the j-th image is a high light image or not. Specifically, it is determined as the high light image if Cnt ≥ β. β is a threshold value for specifying how many pixels should exist for the total number of pixels to determine it as the high light image, that is, a threshold value of an image-captured state to determine if the image is satisfactory or not. In the present embodiment, β is set as β = 0.5 x ISX x ISY, for example, that is, to the number of pixels of 50% in the total number of pixels. In the case of Cnt ≥ β, the program goes on to Step S 15, while in the case of Cnt < β, the program goes on to Step S16. Steps S12 and S 13 constitute a feature value calculation process or feature value calculation means for calculating a feature value on the basis of the value of a pixel, that is, brightness of the image. Step S14 and Step S15 constitute an image-captured state determining process or image-captured state determining means.

At Step S 15, the j-th image to be processed is determined as a high light image, H[j] = TRUE is set to finish the processing, and the program goes to determination processing for the subsequent (j + 1)-th image.

At Step S16, it is determined if the high light pixel determination at Step S12 has been carried out for all the pixels or not. Specifically, in the case of i < ISX x ISY, 1 is added to the number i specifying the pixel (i = i + 1) at Step S 17, Step S12 to Step S 16 are executed for the next pixel, and the high light pixel determination is carried out for the remaining pixels. In the case of i = ISX x ISY, the processing is finished, and the program goes to the determination processing for the subsequent (j + 1)-th image.

As mentioned above, determination can be made on whether an image to be processed is a high light image or not on the basis of a pixel value of each pixel in the captured image by a series of processing in Steps S 11 to S 17.

In the above, the processing operation for determining the dark space image and the high light image individually has been described, but both images can be determined by a single processing operation. For example, in Step S2 of determination processing of the dark space image described using Fig. 10, instead of determination on whether the i-th pixel belongs to the dark space or not, it is determined if the i-th pixel is a pixel in an appropriate image-captured state or not, that is, the pixel is neither a dark space pixel nor a high light pixel. And at Step S4, instead of determination on whether the j-th image is a dark space image or not, it is determined if the j-th image is in an appropriate image-captured state or not, that is, the image is neither a dark space image nor a high light image. In other words, in the above example, whether the pixel value is equal to or larger or equal to or smaller than a predetermined threshold value is a determination criteria, but whether the pixel value is not equal to or larger and not equal to or smaller than a predetermined threshold value may be also a determination criteria. Specifically, at Step 2, in the case of Td < ri < Th, Td < gi < Th and Td < bi < Th, the i-th pixel is determined as a pixel in an appropriate image-captured state and the program goes on to Step S3, while if not, the program goes on to Step S6. Also, at Step S4, in the case of Cnt > ISX x ISY - α, the j-th image is determined as an image in an appropriate image-captured state, and the program goes on to Step S5, while if not, the program goes on to Step S6. By the above processing operation, a dark space image or high light image and inappropriate for observation can be detected in a single processing operation. In the above example, if TRUE is set to the flag D[j], it indicates that the j-th image is determined as an image in an appropriate image-captured state. On the other hand, if FALSE is set to the flag D[j], it indicates that the j-th image is determined as a dark space image or high light image.

Next, a processing operation for determining a foreign substance image will be described using Fig. 12. Typical foreign substances not relating to diagnosis is residues such as feces in a colon. Usually, in a lower digestive tract inspection, pre-treatment is performed for excretion of feces or the like in a colon by taking a meal with less dietary fiber in a day before or the day of the inspection or by taking a large amount of laxative. However, there are cases that feces or the like are not fully excreted but remain in a colon, which makes the residue. Such residue is also generated by deterioration of a peristaltic motion due to aging or the like. Since usual endoscopic inspections are carried out in a hospital, the inspection is performed while nurses or the like are checking the excretion state of the subject. On the other hand, the pretreatment of the digestive tract inspection using the capsule type endoscope 3 is left to the subject in many cases as compared with normal endoscopic inspections, and there is a higher possibility that residues are generated due to incomplete excretion or the like.

In the present embodiment, determination on whether residues exist in an image or not, that is, determination of a foreign substance image is made on the basis of the tone of the feces. First at Step S21, i is initialized to 1, which indicates the number specifying a pixel in the captured image signal data of the j-th image (j is an integer equal to or larger than 1) to be processed. Also, a counter Cnt for counting the number of pixels determined that a foreign substance is captured is initialized to 0. Moreover, a flag A1[j] indicating the determination result on whether the j-th image is a foreign substance image or not is initialized to FALSE. The number i specifying the pixel and the counter Cnt take integer values not less than 1 and not more than ISX x ISY, and either one of TRUE indicating determination as a foreign substance image or FALSE indicating determination as not a foreign substance image is set to a value of the flag A1[j].

Next, at Step S22, a parameter indicating a tone of the i-th pixel Pi is calculated. Suppose that the value of the i-th pixel in each of the R image, the G image and the B image is ri, gi and bi, the parameter indicating the tone in a pixel Pi can be represented by any one or a combination of two of ri, gi and bi. Here, in the values of the pixels ri, gi and bi in an image obtained by capturing a living mucous surface by the capsule type endoscope 3, the value of ri is larger than the value of gi and the value of bi in general. This is because the tone of the living mucous is largely affected by hemoglobin, and hemoglobin has a characteristic that it hardly absorbs but reflects light in a long wavelength band forming the R image and absorbs light in a medium to short wavelength band forming the G image and the B image. On the other hand, the residue by feces is yellow or ocher in general due to influence of digestive juice such as bile or the like. That is, in the tone in the pixel Pi, the value of gi and the value of ri are larger than the value of bi. That is, the tone of the residue by feces has a relatively larger gi value as compared with the tone of the living mucous surface. Thus, it is only necessary to determine whether the pixel Pi is a pixel capturing an image of the living mucous surface or a pixel capturing an image of a foreign substance such as feces on the basis of the tone of the pixel, and specifically, parameters indicating the tone on the basis of the ratio of ri to gi and bi in the pixel Pi may be used. As the parameters indicating the tone on the basis of the above ratio in the pixel Pi, gi/ri, bi/ri, log (gi/ri), log (bi/ri), atan (gi/ri), atan (bi/ri) and the like may be used. However, atan indicates $\tan^{-1}$. In the present embodiment, atan (gi/ri) and atan (bi/ri) are used as parameters indicating the tone, which are represented as a parameter x and a parameter y, respectively.

As a method for calculating the parameter x and the parameter y in the pixel Pi at Step S22, the values of ri, gi an bi in the pixel Pi may be directly substituted in an equation of the parameter x and the parameter y, that is, atan (gi/ri) and atan (bi/ri) for calculation. In the present embodiment, v1 taking an arbitrary integer value in a range of $0 \leq v1 \leq 255$ and v2 taking an arbitrary integer value in a range of $0 \leq v2 \leq 255$ are defined. And a value of atan (v1/v2) for the arbitrary v1 and v2 is calculated in advance and prepared in a two-dimensional array table as shown in Fig. 13. When the parameter x is to be calculated, the value of gi in the pixel Pi is set as v1, the value of ri is set as v2 and atan (v1/v2) corresponding to them is searched in the array table so that a numeral value indicated in the applicable place in the table is taken as the value of the parameter x. For example, if the value of gi in the pixel Pi is 0 and the value of ri is 3, vi = 0 and v2 = 3. In the array table in Fig. 13, atan (v1/v2) corresponding to them is the fourth row from the top and the value is 0. Thus, the value of the parameter x in this case is 0. When the parameter y is to be calculated, the value of bi in the pixel Pi is set as v1, the value of ri is set as v2 and atan (v1/v2) corresponding to them is searched in the array table so that a numeral value indicated in the applicable place in the table is taken as the value of the parameter y. Incidentally, atan (v1/v2) takes a real number value in a range of $0 \leq$ atan (v1/v2) $< 90$. In the present embodiment, for simplification of the processing, the range is divided into 90 parts and discrete approximated values are applied. For example, by rounding off the first decimal point, the value of atan (v1/v2) is approximated to an integer value from 0 to 89. For example, if the value of bi in the pixel Pi is 255 and the value of ri is 254, v1 = 255 and v2 = 254. In the array table in Fig. 13, atan (v1/v2) corresponding to them is the second row from the bottom, and the value is 45.112. Thus, the value obtained by rounding off the first decimal point of 45.112, which is 45, is made as the value of the parameter y. Step S22 constitutes a tone extraction process.

Next, at Step S23, using parameters indicating the tone of the i-th pixel Pi, it is determined if the pixel Pi is a pixel capturing an image of a foreign substance. In the determination of a foreign substance pixel in the present embodiment, an area map prepared in advance prior to the determination of the foreign substance pixel is used, in which distribution areas of foreign substance pixels are defined. The area map is a two-dimensional diagram with the parameter x as the x-axis and the parameter y as the y-axis, and distribution areas are defined, respectively, on the basis of positions where the pixel determined as a foreign substance and the pixel determined as the living mucous surface in many images having been captured are plotted. The residues such as feces have a strong yellow tone, and the value of gi takes a relatively large value. Thus, the foreign substance image is defined to distribute in an area as shown in an area (1) in Fig. 14, for example. Also, the living mucous surface is defined to distribute in an area as shown in an area (2) in Fig: 14, for example. The x-axis and the y-axis are divided into 90 parts, respectively, using ninety discrete values which can be taken as the values of the parameter x and the parameter y, by which the area map is divided into sections of 90 x 90. Moreover, the following values are given to each of the sections. That is, 1 is given to the section included in the area (1), 2 is given to the section included in the area (2), and 0 is given to the section not included in either of them. It is only necessary that the value given to the section not included in either of the areas is not 1, and 2 may be given, for example.

The determination on whether the pixel Pi is a pixel capturing an image of a foreign substance or not is made based on whether a positional coordinate determined by the value of the parameter x and the value of the parameter y indicating the tone of the pixel Pi obtained at Step S22 in the above area map is included in the distribution area of the foreign substance pixel, that is, belongs to the section to which 1 is given as the value. Therefore, the boundary of the distribution areas constitutes the threshold of the tone. If it belongs to the section to which 1 is given as the value, the pixel Pi is determined as the pixel capturing an image of a foreign substance, and the program goes on to Step S24. If it belongs to the section to which a value other than 1 is given, the pixel Pi is determined as a pixel not capturing a foreign substance, and the program goes on to Step S27. Steps S22, S23, and S24 constitute a feature value calculation process or feature value calculation means for calculating a feature value on the basis of the tone of the image.

At Step S24, the value of Cnt is incremented by 1. Then, at Step S25, it is determined if the j-th image is a foreign substance image or not. Specifically, it is determined as the foreign substance image, if $Cnt \geq \gamma$. $\gamma$ is a threshold value for specifying how many pixels should exist for the total number of pixels to determine it as the foreign substance image, that is, a threshold value of an image-captured state to determine if the image is satisfactory or not. In the present embodiment, $\gamma$ is set as $\gamma = 0.5 \times ISX \times ISY$, for example, that is, to the number of pixels of 50% in the total number of pixels. In the case of $Cnt \geq \gamma$, the program goes on to Step S26, while in the case of $Cnt < \gamma$, the program goes on to Step S27.

At Step 26, the j-th image to be processed is determined as the foreign substance image, processing is finished as A1 [j] = TRUE, and the program goes to determination processing for the subsequent (j + 1)-th image. Steps S25 and S26 constitute an image-captured state determining process or image-captured state determining means.

At Step S27, it is determined if the foreign substance pixel determination at Step S23 has been carried out for all the pixels or not. Specifically, in the case of i < ISX x ISY, 1 is added to the number i specifying the pixel (i = i + 1) at Step 28, Step S22 to Step S27 are executed for the next pixel and the foreign substance pixel determination is carried out for the remaining pixels. In the case of i = ISX x ISY, the processing is finished, and the program goes to the determination processing for the subsequent (j + 1)-th image.

As mentioned above, by a series of processing in Steps S21 to S28, determination can be made if the image to be processed is a foreign substance image or not on the basis of the pixel value of each pixel of the captured image.

In the above, the processing operation for determining the dark space image, high light image and foreign substance image individually has been described, but these three kinds of inappropriate images can be determined by a single processing operation. One example of the processing operation for determining the above three kinds of inappropriate images will be described using Fig. 15.

First, at Step S31, i is initialized to 1, which indicates the number specifying a pixel in the captured image signal data of the j-th image (j is an integer equal to or larger than 1) to be processed. Also, a counter CntD for counting the number of pixels determined as a dark space, a counter CntH for counting the number of pixels determined as a high light, and a counter CntA for counting the number of pixels determined that a foreign substance is captured are initialized to 0. Moreover, a flag N[j] indicating the determination result on whether the j-th image is an inappropriate image or not is initialized to FALSE. The number i specifying the pixel and the counters CntD, CntH and CntA take integers not less than 1 and not more than ISX x ISY, and either one of TRUE indicating determination as the inappropriate image or FALSE indicating determination as not the inappropriate image is set to a value of the flag N[j].

Next, at Step S32, it is determined if the i-th pixel belongs to a dark space or not. Since the processing at Step S32 is the same as the processing at Step S2, description of the detail of the processing will be omitted. If the i-th pixel is determined as a pixel belonging to a dark space, the program goes on to Step S33, while if the i-th pixel is determined as a pixel not belonging to a dark space, the program goes on to Step S35, where high light pixel determination is carried out.

At Step S33, the value of CntD is incremented by 1. Then, at Step S34, it is determined if the j-th image belongs to a dark space or not. Since the processing at Step S34 is the same as the processing at Step S4, description of the detail of the processing will be omitted. If the j-th image is determined as a dark space image, the program goes on to Step S42, while if the j-th image is determined as not a dark space image, the program goes on to Step S35.

At Step S35, it is determined if the i-th pixel belongs to a high light pixel or not. Since the processing at Step S35 is the same as the processing at Step S12, description of the detail of the processing will be omitted. If the i-th pixel is determined as a pixel belonging to a high light image, the program goes on to Step S36, while if the i-th pixel is determined as a pixel not belonging to a high light pixel, the program goes on to Step S38, where foreign substance pixel determination is carried out.

At Step S36, the value of CntH is incremented by 1. Then, at Step S37, it is determined if the j-th image is a high light image or not. Since the processing at Step S37 is the same as the processing at Step S14, description of the detail of the processing will be omitted. If the j-th image is determined as a high light image, the program goes on to Step S42, while if the j-th image is determined as not a high light image, the program goes on to Step S38.

At Step S38, parameters indicating the tone of the i-th pixel Pi are calculated. Since the processing at Step S38 is the same as the processing at Step S22, description of the detail of the processing will be omitted. Then, at Step S39, using the parameters indicating the tone of the i-th pixel Pi, it is determined if the pixel Pi is a pixel capturing an image of a foreign substance. Since the processing at Step S39 is the same as the processing at Step S23, description of the detail of the processing will be omitted. If the i-th pixel Pi is determined as a pixel capturing an image of a foreign substance, the program goes on to Step S40, while if the i-th pixel Pi is determined as not a pixel capturing an image of a foreign substance, the program goes on to Step S43.

At Step S40, the value of CntA is incremented by 1. Then, at Step S41, it is determined if the j-th image is a foreign substance image or not. Since the processing at Step S41 is the same as the processing at Step S25, description of the detail of the processing will be omitted. If the j-th image is determined as a foreign substance image, the program goes on to Step S42, while the j-th image is determined as not a foreign substance image, the program goes on to Step S43.

At Step 42, the j-th image to be processed is determined as an inappropriate image, processing is finished as N[j] = TRUE, and the program goes to determination processing for the subsequent (j + 1)-th image.

At Step S43, it is determined if the inappropriate pixel determination has been carried out for all the pixels or not. Specifically, in the case of i < ISX x ISY, 1 is added to the number i specifying the pixel (i = i + 1) at Step 44, and the inappropriate pixel determination is carried out for the remaining pixels. In the case of i = ISX x ISY, the processing is finished, and the program goes to the determination processing for the subsequent (j + 1)-th image.

As mentioned above, by a series of processing in Steps S31 to S44, determination can be made if the image to be processed is an inappropriate image classified into any of a dark space image, a high light image and a foreign substance image on the basis of the pixel value of each pixel of the captured image. Determination of belonging has been made in the order of a dark space pixel, a high light pixel and a foreign substance pixel, but the order of determination is not limited to this but the determination may be started from the foreign substance pixel or the high light pixel. Also, the determination of a dark space pixel, a high light pixel and a foreign substance pixel may be made in a single step.

Next, a processing operation for determining an excessively close-up image will be described using Fig. 16. If the capsule type endoscope 3 gets excessively close to or contact with a living mucous, an entire captured image becomes red, yellow or the like. Even not in contact, in the case of excessively proximity to the living mucous, a captured image is defocused, and discovery of lesions or observation findings of a vessel image might become difficult to be obtained.

In the present embodiment, an average value and a standard deviation of tone of an entire image are made as feature values, and determination of the excessively close-up image is made on the basis of these feature values. First, at Step S51, a flag A2[j] indicating the determination result on whether the j-th image (j is an integer equal to or larger than 1) to be processed is an excessively close-up image or not is initialized to FALSE. Either one of TRUE indicating determination as an excessively close-up image or FALSE indicating determination as not an excessively close-up image is set to a value of the flag A2[j].

Next, at Step S52, determination is made for all the pixels on whether a pixel in the j-th image to be processed is a dark space pixel or high light pixel. For the determination of the dark space pixel at this step, the determination processing at step S2 in Fig. 10 may be carried out for all the pixels Pi in a range of $1 \leq i \leq$ ISX x ISY. Also, for the determination of the high light pixel at this step, the determination processing at step S12 in Fig. 11 may be carried out for all the pixels Pi in the range of $1 \leq i \leq$ ISX x ISY.

Subsequently, at Step S53, values of gi/ri and bi/ri are calculated for all the pixel except the pixels determined as the dark space pixel or high light pixel at step S52, and an average value and a standard deviation of the calculation target pixels are calculated. In the present embodiment, four values of the average value of gi/ri, standard deviation of gi/ri, average value of bi/ri and standard deviation of bi/ri are used as feature values for identification/classification, and determination is made on an excessively close-up image.

Then, at Step S54, the images to be processed are identified/classified using a known linear discriminant function. In the identification/classification, a plurality of classification called as classes are defined in advance and a linear discriminant function is generated using the feature values calculated from known data called as teacher data and classified into any of these plurality of classes, and by inputting the feature value of data to be classified into this linear discriminant function, the target data is classified into any of the classes, that is, a threshold value of image-captured state determining whether the image is satisfactory or not. As a method for identification/classification, an identifier such as neural network may be used other than the linear discriminant function.

In the present embodiment, two images: an image obtained by normally capturing an image of a living mucous surface and an excessively close-up image obtained by capturing excessively close to or in contact with the living mucous surface are defined as the classes, and a linear discriminant function is generated using hundred images classified into each class as teacher data, for example. Since the entire image becomes red or yellow in the excessively close-up image, the excessively close-up image class may be further divided into two classes of a red-tone excessively close-up image class and a yellow-tone excessively close-up image class on the basis of its average tone, which makes three classes together with a normal image class in order to improve accuracy of identification/classification. When the entire image becomes red in an excessively close-up image, the values of gi and bi become smaller than the value of ri, and an average value of gi/ri and an average value of bi/ri are both small, while when the entire image is yellow, the average value of gi/ri is larger than the average value of bi/ri. Also, since excessive proximity of the capsule type endoscope 3 to the living mucous surface makes the image defocused or its contact with the living mucous surface makes the entire image blurred, the standard deviation of gi/ri and the standard deviation of bi/ri are both small values. The linear discriminant function classifies images to be processed to any of the classes on the basis of a difference in distribution of these feature values in each class. Steps S52, S53 and S54 constitute a feature value calculating process or feature value calculating means.

Next, at step S55, on the basis of the identification/classification result at Step S54, it is determined if the image to be processed is an excessively close-up image or not. At Step S54, when the image to be processed is classified into the excessively close-up image class or any of the red-tone excessively close-up class and the yellow-tone excessively close-up class divided from the excessively close-up class, the image to be processed is determined as an excessively close-up image, and the program goes on to Step S56. At Step S54, if the image to be processed is classified into a normal image class, the image to be processed is determined as not an excessively close-up image, the processing is finished, and the program goes to determination processing for the subsequent (j + 1)-th image. At Step S56, the processing is finished as A2[j] = TRUE, and the program goes to determination processing for the determination processing for the subsequent (j + 1)-th image. Steps S55 and S56 constitute an image-captured state determining process or image-captured state determining means.

As mentioned above, by identification/classification through calculation of feature values from a pixel value of each pixel of the captured image in a series of processing of Steps S51 to S56, it can be determined if the image to be processed is an excessively close-up image or not.

Next, a processing operation for determining other observation inappropriate images will be described using Fig. 17. There might be some places where water is accumulated in a digestive tract, and if the capsule type endoscope 3 is submerged in such a place, images in which the living mucous surface cannot be observed might be captured. Also, if the capsule type endoscope 3 is moved at a high speed in the digestive tract or makes a rapid peristaltic motion due to pulsation or the like, an image in which the visual field flows instantaneously might be captured. Most of these images are defocused, and observation of a vessel image or structure of the living mucous surface is difficult.

In the present embodiment, other observation inappropriate images are determined on the basis of a frequency component amount included in the image. Since the image reflecting the structural component of the living mucous surface the most is the G image, only the G image is used for determination of the image.

First, at step S61, a flag A3[j] indicating the determination result that the j-th image (j is an integer equal to or larger than 1) to be processed is one of other observation inappropriate image or not is initialized to FALSE. For the value of the flag A3[j], either of TRUE indicating determination as one of other observation inappropriate images or FALSE indicating determination as not one of other observation inappropriate images is set.

Next, at Step S62, determination is made on whether a pixel in the j-th image to be processed is a dark space pixel or high light pixel for all the pixels, and the location of the pixel determined as the dark space pixel or high light pixel is stored. For the determination of the dark space pixel at this step, the determination processing at step S2 in Fig. 10 may be carried out for all the pixels Pi in a range of $1 \leq i \leq ISX \times ISY$ Also, for the determination of the high light pixel at this step, the determination processing at step S12 in Fig. 11 may be carried out for all the pixels Pi in the range of $1 \leq i \leq ISX \times ISY$ The location of the pixel determined as the dark space pixel or high light pixel is stored as follows. That is, first, a two-dimensional array area with the size of ISX x ISY is ensured in a memory in advance, and a value of an array element corresponding to a coordinate position of the pixel determined as the dark space pixel is set to 1, a value of an array element corresponding to a coordinate position of the pixel determined as the high light pixel to 2, and a value of an array element corresponding to a coordinate position of the pixel determined as one of other pixels to 0.

Then, at Step S63, a band pass filtering is applied to the entire image. As the band pass filtering, a known digital filter (FIR filter) is used, and only the frequency band component constituting the living mucous surface structure such as a vessel image is extracted. The frequency characteristic of the digital filter used in the present embodiment has a peak at $f = \pi/3$ to the highest frequency $\pi$ in the image and restricts a low-frequency component and a high-frequency component. Step S63 constitutes a filtering process.

Next, at Step S64, on the basis of the position information of the dark space pixel and the high light pixel determined and stored at step S62, a modification processing is executed for eliminating an influence on a band pass filtering result caused by too dark or too bright pixels to the band pass filtering result obtained at Step S63. Since the S/N ratio is deteriorated in the dark space pixel, that is, in an extremely dark pixel, a component caused by a noise has a larger effect on the band pass filtering result than the component caused by the living mucous surface structure. Thus, the component of the living mucous surface structure cannot be properly extracted in the dark space image.

Also, the high light pixel is an extremely bright pixel, and since the pixel value of a pixel at the peripheral boundary of the high light area is rapidly changed, it causes a large fluctuation in the band pass filtering result. For example, suppose that a substantially oval high light area H1 exists in the vicinity of the center of the image, as shown in Fig. 19A, and the value of the pixel located on an axis a-a' in the horizontal direction of the image passing through the high light area H1 shows a profile as in Fig. 19B. If the band pass filtering is applied to this image, an affected area H2 is generated at the peripheral boundary of the high light area as shown in Fig. 19C, and a rapid fluctuation is caused in the profile in the horizontal direction in an extremely short distance in the affected area H2 as shown in Fig. 19D. The spread degree of the affected area H2 depends on the digital filter size used in the band pass filtering, and if the filter size is N x N, it is |N/2|. Here, || is a Gauss symbol, which means that the figures after the decimal point is rounded. The band filtering in the present embodiment has a characteristic that the amplitude of a direct current component is 0, and thus, it can take a negative value in the processing result.

The modification processing for the band pass filtering result will be carried out as follows. First, in the two-dimensional array area in which the positions of the dark space pixel and the high light pixel are stored, by applying the known expansion processing to the high light pixel area, the value of the array element corresponding to the position coordinate of the pixel corresponding to the affected area H2 generated by high light is substituted by 2, indicating the high light pixel. Next, in the two-dimensional array area after application of the expansion processing, the value of the extracted component obtained by applying the band pass filtering to the pixel to which 1 or 2 is given as the value of the array element is substituted by 0. Also, the number of pixels whose values of the extracted components are substituted by 0 is stored by the counter Cnt. By the above processing, influence of the dark space pixels and the high light pixels are eliminated from the result obtained by the band pass filtering and modified to those extracting only the frequency band components constituting the living mucous surface structure such as a vessel image.

Next, at Step S65, on the basis of the structural component extraction result obtained by the processing up to Step S64, the structural component feature value is calculated. In the present embodiment, a square mean value of the extracted structural component is defined as the structural component feature value. The square mean value $\mu$ is generally called as a frequency power, and the more structural component is extracted from the pixel, in other words, the higher the frequency component is, the higher value the frequency power takes, and it is calculated by the following equation (1):

[Equation 1]

$$\mu = \left\{ \sum_{j=1}^{ISY} \sum_{i=1}^{ISX} h^2(i,j) \right\} / \left\{ (ISX \times ISY) - Cnt \right\}$$

In the equation (1), h (i, j) is a structural component extraction result of each pixel after the dark space pixels, the high light pixels and the pixels affected by high light are eliminated, and Cnt is the number of pixels for which the value of the extracted component is substituted by 0 at Step S64. Step S65 constitutes a frequency power calculating process. Also, Steps S63 to Step S65 constitute a frequency extracting process. Steps S62 to S65 constitute a feature value calculating process or feature value calculating means. Particularly, Steps S64 and 65 constitute the feature value calculating process or feature value calculating means for calculating a feature value on the basis of the frequency component amount or the structural component of an image.

Next, at Step S66, on the basis of the structural component feature value obtained at Step S65, it is determined if the image to be processed is one of other observation inappropriate images or not. Specifically, if $\mu \leq Tf$, the image to be processed is determined as an image with less structural component and defocused, that is, one of other observation inappropriate images, and the program goes on to Step S67. In the case of $\mu > Tf$, the image to be processed is determined as not one of other observation inappropriate images, the processing is finished, and the program goes to the determination processing for the subsequent (j + 1)-th image. Here, Tf is a threshold value determined in advance for determining other observation inappropriate images, that is, a threshold value of an image-captured state to determine if the image is satisfactory or not. At Step S67, the processing is finished as A3[j] = TRUE, and the program goes on to the determination processing for the subsequent (j + 1)-th image. Steps S66 and S67 constitute an image-captured state determining process or image-captured state determining means.

As mentioned above, only the frequency band component constituting the living mucous surface structure included in the captured image is extracted by the series of processing of Steps S61 to S67, and determination can be made on whether the mage to be processed is one of other observation inappropriate images or not on the basis of the structural component feature value calculated from the extraction result.

In the present embodiment, as a method for extracting the frequency band component constituting the living mucous surface structure, the band pass filtering using a digital filter is applied, but a known edge detection filter such as a Laplacian may be applied. Also, a square mean value of the extracted structural component is used as the structural component feature value, but a standard deviation or distribution of a pixel value in the G image may be used. The smaller the living mucous surface structure is, the smaller value the standard deviation or distribution takes.

Also, the above series of processing for determining other observation inappropriate images may be used as the processing for determining the excessively close-up image mentioned above. In the present embodiment, the excessively close-up images and other observation inappropriate images are classified into different image-captured states, but since both images are defocused and can be defined as images with little or no living mucous surface structure, they can be determined all at once by the above processing. In this case, high speed processing can be promoted.

Next, a method for determining an inappropriate image in the capsule type endoscope device 1 will be described using Fig. 20. Determination of the inappropriate image is to determine which of the inappropriate images classified into five classes the image to be processed belongs to, and the determination processing of the above-mentioned five kinds of inappropriate images is used for the processing. In other words, the processing comprised by each step shown in Fig. 20 constitutes classifying means for classifying them on the basis of the image-captured state of the respective images.

First, at Step S71, a flag C[j] indicating the determination result on whether the j-th (j is an integer equal to or larger than 1) image to be processed is an inappropriate image or not is initialized to 0. For the value of the flag C[j], a value of any of 1 to 4 indicating determination as an inappropriate image or 0 indicating determination not as an inappropriate image is set. Next, at Step S72, it is determined if the image to be processed is a dark space image or not. For the processing at Step S72, the series of processing at Steps S1 to S7, which is the determination processing of the dark space image described using Fig. 10, is used.
If the determination processing result of the dark space image is D[j] = TRUE, that is, if the image to be processed is determined as a dark space image, the processing of an inappropriate image is finished as C[j] = 1 at the subsequent Step S73, and the program goes on to the determination processing for the subsequent (j + 1)-th image. If the determination processing result of the dark space image is D[j] = FALSE, that is, if the image to be processed is determined as not a dark space image, the program goes on to Step S74.

At Step S74, it is determined if the image to be processed is a high light image or not. For the processing at Step S74, the series of processing at Steps S11 to S17, which is the determination processing of the high light image described using Fig. 11, is used. If the determination processing result of the high light image is H[j] = TRUE, that is, if the image to be processed is determined as a high light image, the processing of an inappropriate image is finished as C[j] = 2 at the subsequent Step S75, and the program goes on to the determination processing for the subsequent (j + 1)-th image. If the determination processing result of the high light image is H[j] = FALSE, that is, if the image to be processed is determined as not a high light image, the program goes on to Step S76.

At Step S76, it is determined if the image to be processed is a foreign substance image or not. For the processing at Step S76, the series of processing at Steps S21 to S28, which is the determination processing of the foreign substance image described using Fig. 12, is used. If the determination processing result of the foreign substance image is A1[j] = TRUE, that is, if the image to be processed is determined as a foreign substance image, the processing of an inappropriate image is finished as C[j] = 3 at the subsequent Step S77, and the program goes on to the determination processing for the subsequent (j + 1)-th image. If the determination processing result of the foreign substance image is A1[j] = FALSE, that is, if the image to be processed is determined as not a foreign substance image, the program goes on to Step S78.

At Step S78, it is determined if the image to be processed is an excessively close-up image or not. For the processing at Step S78, the series of processing at Steps S51 to S56, which is the determination processing of the excessively close-up image described using Fig. 16, is used. If the determination processing result of the excessively close-up image is A2[j] = TRUE, that is, if the image to be processed is determined as an excessively close-up image, the processing of an inappropriate image is finished as C[j] = 4 at the subsequent Step S79, and the program goes on to the determination processing for the subsequent (j + 1)-th image. If the determination processing result of the excessively close-up image is A2[j] = FALSE, that is, if the image to be processed is determined as not an excessively close-up image, the program goes on to Step S80.

At Step S80, it is determined if the image to be processed is one of other observation inappropriate images or not. For the processing at Step S80, the series of processing at Steps S61 to S67, which is the determination processing of an other observation inappropriate image described using Fig. 17 is used. If the determination processing result of other observation inappropriate images is A3[j] = TRUE, that is, if the image to be processed is determined as one of other observation inappropriate images, the processing of an inappropriate image is finished as C[j] = 5 at the subsequent Step S81, and the program goes on to the determination processing for the subsequent (j + 1)-th image. If the determination processing result of other observation inappropriate images is A3[j] = FALSE, that is, if the image to be processed is determined as not one of other observation inappropriate images, the processing of an inappropriate image is finished, and the program goes on to the determination processing for the subsequent (j + 1)-th image.

The determination processing of an inappropriate image as mentioned above is implemented as software program and executed at the terminal device 7 in the present embodiment. The terminal device 7 takes in a series of images captured by the capsule type endoscope 3 and recorded in the external device 5 through the cradle 6. At this image taking-in, the determination processing of an inappropriate image shown at Steps S71 to S81 is executed and the determination result is stored in association with the taken-in image. Using the stored determination result, the inappropriate images are eliminated from the series of images taken into the terminal device 7, while only the remaining images appropriate for observation and diagnosis are displayed on the display 8c so that observation efficiency can be improved.

An image display method for eliminating the inappropriate images from the series of images and displaying the remaining images on the display 8c will be described using Fig. 21. In the present embodiment, the images from the first to the last taken into the terminal device 7 are displayed as still images according to the order of taken-in images. Alternately, they are displayed continuously as a slide show. The terminal device 7 includes a central processing unit (CPU), not shown, and a memory for executing the processing, which will be described below. Therefore, the terminal device 7 has a program for executing the processing, which constitutes control means along with the program, and controls the following processing relating to the display 8c, which is display means.

The terminal device 7 first initializes j to 1, which is a number identifying an image to be processed and indicates the number in the order by which the image is taken into the terminal device at Step S91, and the first taken-in image is made as an image to be processed. Next, at Step S92, the value of C[j] recorded in association with the j-th image is referred to from the determination result of the inappropriate image, described using Fig. 20. If C[j] = 0, that is, the j-th image is determined not as an inappropriate image, the program goes on to Step S93, the j-th image is displayed on the display 8c, and the program further goes on to Step S94. In the case of C[j] ≠ 0, that is, the j-th image is determined as an inappropriate image, the j-th image is not displayed on the display 8c but the program goes on to Step S94.

At Step S94, in order that the image taken into the terminal device 7 subsequently to the j-th image is made as a target to be processed, j = j + 1 is set. Then, at Step S95, it is determined if the above-mentioned image display availability determination and image display processing have been executed for all the images taken into the terminal device 7 or not. For example, suppose that the total number of images taken into the terminal device 7 is N, in the case of j ≤ N, the program returns to Step S92, where the similar processing is carried out for the remaining images. In the case of j > N, the processing is finished. The above Steps S91 to S95 constitute display control means and a display controlling process.

By the above processing, at the terminal device 7, inappropriate images are eliminated from the images captured by the capsule type endoscope 3 and taken in through the external device 5 and only the images appropriate for observation and diagnosis can be displayed on the display 8c.

According to the capsule type endoscope device 1 and the image processing method of the present embodiment, images inappropriate for observation and diagnosis can be determined in this way. Also, by not displaying the images determined as inappropriate, time required for observation and diagnosis can be reduced and work efficiency can be improved.

In order to prevent oversight of a lesion by any chance, it might be necessary to check the image determined as an inappropriate image on the display 8c or the like. In order to handle this issue, it is possible to add a function to list/display inappropriate images in a lump sum or by categorized type in an observation program operating at the terminal device 7. For example, if the observation program is provided with a window and GUI (graphic user interface), a button such as a dark space image display button is provided for displaying a list of inappropriate images on the window and GUI, and when the button is clicked by the mouse 8b, all the inappropriate images or inappropriate images belonging to the classification specific to the dark space image are shrunk and displayed in a list. By this, the inappropriate images can be checked efficiently.

Also, in the present embodiment, determination of an inappropriate image is made while handling all the pixels in the image equally, but determination may be made by weighting pixels at the center area in an image which can obtain favorable image-captured conditions than the pixels in the peripheral area, for example. Specifically, a section located at the center when the entire image is divided into nine parts is set as an image center area, and at Step S2 of the determination processing of the dark space image, for example, determination conditions of a dark space pixel may be made more strict by setting a threshold value for determining if the pixel belonging to the image center area is a dark space pixel or not to a higher value such as 1.1 times of the threshold value for the pixels belonging to the other areas. Alternately, at Step S2 of the determination processing of the dark space image, if the pixel belonging to the image center area is determined as a dark space pixel, an increment of Cnt counting the dark space pixel may be weighted by setting it to 1.5 against the value of pixels belonging to the peripheral areas at 1 at the subsequent Step S3. Also, weighing may be made by a two-dimensional normal distribution function or the like having a peak at the image center area.

Moreover, in the present embodiment, determination of an inappropriate image is made for an image captured by the capsule type endoscope 3 and taken into the terminal device 7, but the determination of an inappropriate image may be made for images scaled down by pixel skipping or the like, for example. Also, determination of an inappropriate image is made using all the pixels in an image in the present embodiment, but the determination of an inappropriate image may be made by using pixels sampled from the image as appropriate. In this case, it is possible to make the determination of an inappropriate image by sampling more pixels from the pixels belonging to the image center area from which a favorable image-captured condition can be obtained than the pixels belonging to the peripheral area of the image. Furthermore, the determination of an inappropriate image and the determination of availability of display on the display 8c are made at the terminal device 7 in the present embodiment, but these determinations may be made at the external device 5. Also, in the present embodiment, images are categorized to those appropriate for observation and diagnosis and those not, but appropriateness for observation and diagnosis is continuously evaluated and stored according to the proportion of the dark space pixels, for example, so that it can be referred to as necessary. In this case, it is possible that a threshold value for determining if the image is to be stored or not is set at the terminal device 7, and storage or not is determined by comparing the evaluation value with the threshold value.

(Second embodiment)

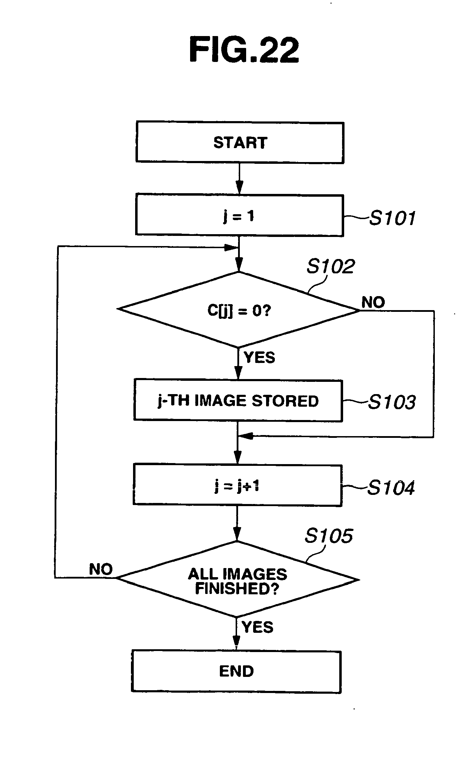

Next, a second embodiment of the present invention will be described using Fig. 22. Fig. 22 is a flowchart for explaining an image storing operation at the terminal device 7. The image processing method according to the present embodiment is to detect an image inappropriate for observation and diagnosis from a series of images obtained from the capsule type endoscope 3 so that the image determined as an inappropriate image is not to be stored in a large capacity memory device (usually, a hard disk drive is used) as output means incorporated in the terminal body 9. Thus, it becomes possible to reduce data amount stored in the memory device so as to lower costs of the device or to shorten time required for observation. Since the entire configuration of the capsule type endoscope device 1 is the same as that of the first embodiment, the same reference numerals are given to the same configuration and the description will be omitted. Also, since the determination processing of various inappropriate images in the present embodiment is the same as the processing in the first embodiment described using Figs. 10 to 20, the same reference numerals are given to the same configuration and the description will be omitted. Here, only the image storing method for eliminating inappropriate images from a series of images and storing the remaining images in a memory device to be a characteristic of the present embodiment will be described.

In the present embodiment, similar to the first embodiment, the determination processing of a series of inappropriate images is implemented as software program and executed at the terminal device 7. In the present embodiment, the terminal device 7 takes in a series of images captured by the capsule type endoscope 3 and recorded in the external device 5 through the cradle 6. At this taking-in of the images, the determination processing of an inappropriate image shown in Steps S71 to S81 described using Fig. 20 is executed and the determination results and the taken-in images are stored in association with each other. And by using the stored determination results, inappropriate images are eliminated from the series of images taken into the terminal device 7 and only the remaining images appropriate for observation and diagnosis are stored in the memory device of the terminal body 9. That is, the data amount stored in the memory device can be reduced so as to lower the device cost, and observation efficiency can be improved.

The image storing method for eliminating the inappropriate images from the series of images and storing the remaining images in the terminal device 7 will be described using Fig. 22. In the present embodiment, similar to the first embodiment, the images from the first to the last taken into the terminal device 7 are displayed as still images according to the order of images taken in or they are displayed continuously as a slide show. The terminal device 7 includes a central processing unit (CPU), not shown, and a memory and executes processing, which will be described below. Therefore, the terminal device 7 has a program for executing the processing, which constitutes control means along with the program, and controls the following processing relating to the memory device (not shown) such as a hard disk as storing means.

At execution of the program, first at Step S101, the terminal device 7 initializes j to 1, which is the number identifying an image to be processed and indicating the order of the image taken into the terminal device 7 so as to make the first taken-in image as a target to be processed. Next, from the determination result of an inappropriate image described at Step S102 using Fig. 20, the value of C[j] recorded in association with the j-th image is referred to. In the case of C[j]=0, that is, if the j-th image is determined as not an inappropriate image, the program goes on to step S103, the j-th image is stored in a large-capacity memory device (usually, a hard disk drive is used) incorporated in the terminal body 9, and the program goes on to Step S104. In the case of C[j] ≠ 0, that is, if the j-th image is determined as an inappropriate image, the j-th image is not stored in the large-capacity memory device incorporated in the terminal body 9 but the program goes on to Step S104.

At step S 104, in order that the image taken into the terminal device 7 subsequently to the j-th image is made as a target to be processed, j = j + 1 is set. Then, at Step S105, it is determined if the above-mentioned image display availability determination and image storing processing have been executed for all the images taken into the terminal device 7 or not. For example, suppose that the total number of images taken into the terminal device 7 is N, in the case of $j \leq N$, the program returns to Step S102, where the similar processing is carried out for the remaining images. In the case of $j > N$, the processing is finished. The above Steps S101 1 to 105 constitute storage control means and a storage controlling process.

In the above processing, at the terminal device 7, inappropriate images are eliminated from the images captured by the capsule type endoscope 3 and taken in through the external device 5 and only the images appropriate for observation and diagnosis can be stored in the large-capacity memory device incorporated in the terminal body 9.

In this way, with the capsule type endoscope device 1 and the image processing method of the present embodiment, images inappropriate for observation and diagnosis can be determined. Also, with the capsule type endoscope device 1 and the image processing method of the present embodiment, by not storing the images determined as inappropriate, the data amount to be stored in the memory device can be reduced, and the device cost can be lowered. Also, with the capsule type endoscope device 1 and the image processing method of the present embodiment, time required for observation and diagnosis can be reduced, and work efficiency can be improved.

In the present embodiment, the images determined as inappropriate are not stored in the large-capacity memory device incorporated in the device body 9, but the inappropriate images may be stored in the large-capacity memory device after giving them compression processing with a high compression rate. In this case, too, the data amount to be stored in the memory device can be reduced and the device cost can be lowered in the present embodiment.

Also, in the present embodiment, the determination of inappropriate images and the determination of availability of storage in the large-capacity memory device incorporated in the terminal body 9 are made at the terminal device 7, but these determinations may be made at the external device 5.

As mentioned above, the present invention can realize an image processing device which can determine if an image capturing a subject is inappropriate for observation and diagnosis or not and can realize a capsule type endoscope device provided with an image processing device which can determine if an image capturing a subject is inappropriate for observation and diagnosis or not.

**Claims**

**1.** An image processing method comprising:

a feature value calculating process (52, S4; S12, S13; S22, S23, S24; S52, S53, S54; 562, 563, S64, S65) for calculating one or more feature values of each of a plurality of images including a plurality of color signals obtained by capturing an image of a subject, on the basis of brightness and tone and frequency component for each pixel constituting the image;

an image-captured state determining process (S4, S5; S14, S15; S25, S26; S55, S56; 566, S67) for determining whether or not each of the images is an image in an image-captured state inappropriate for observation, from an image-captured state of each of the images on the basis of the calculated feature value by performing determination processes of:

a first determination process for determining a dark space image on the basis of at least one of the number of pixels smaller than a first predetermined value in the image and a proportion of pixels smaller than the first predetermined value in the image, and a feature value on the basis of the brightness calculated in the feature value calculating process,

a second determination process for determining a high light image on the basis of at least one of the number of pixels larger than a second predetermined value in the image and a proportion of pixels larger than the second predetermined value in the image, and a feature value on the basis of the brightness calculated in the feature value calculating process,

a third determination process for determining a foreign substance image on the basis of at least one of the number of pixels having a predetermined tone in the image and a proportion of pixels having the predetermined tone in the image, and a feature value on the basis of the tone calculated in the feature value calculating process,

a fourth determination process for determining an excessively close-up image on the basis of a linear discriminant function using the feature value on the basis of the tone, and

a fifth determination process for determining a defocused image by comparing the feature value on the basis of the frequency with a predetermined threshold value;

the image-captured state determining process performing:

classification of the image in the image-captured state inappropriate for observation as the dark space image based on the determination result of the first determination process,

classification of the image in the image-captured state inappropriate for observation as the high light image based on the determination result of the second determination process,

classification of the image in the image-captured state inappropriate for observation as the foreign substance image based on the determination result of the third determination process,

classification of the image in the image-captured state inappropriate for observation as the excessively close-up image based on the determination result of the fourth determination process, and

classification of the image in the image-captured state inappropriate for observation as the defocused image based on the determination result of the fifth determination process; and

a setting process for setting a flag to indicate as being an image in the image-captured state inappropriate for the observation or an image in an image-captured state appropriate for the observation, for each of the images according to a determining result of the image-captured state determining process.

**2.** The image processing method according to claim 1, wherein the frequency component is extracted by processes including a filtering process (S63) for applying band pass filtering for extracting a frequency component constituting a living mucous surface structural component in the image and a frequency power calculating process (S65) for calculating frequency power of the extracted frequency component; and

in the feature value calculating process, a calculation result by the frequency power calculation process is set as a feature value.

**Patentansprüche**

**1.** Bildverarbeitungsverfahren aufweisend:

einen Merkmalswertberechnungsprozess (S2, S4; S12, S13; S22, S23, S24; 552, 553, 554; S62, S63, 564, S65) zum Berechnen von einem oder mehreren Merkmalswerten von jedem einer Mehrzahl von Bildern enthaltend eine Mehrzahl von Farbsignalen, die erlangt wurden durch Aufnehmen eines Bildes eines Subjekts, auf der Basis von Helligkeits- und Ton- und Frequenzkomponenten von jedem das Bild bildenden Pixeln;

einen Bildaufnahmezustandsbestimmungsprozess (S4, S5; S14, S15; S25, S26; S55, 556; S66, S67) zum Bestimmen ob jedes der Bilder ein Bild in einem Bildaufnahmezustand, der für Beobachtung ungeeignet ist, ist oder nicht, von einem Bildaufnahmezustand jedes der Bilder auf der Basis des berechneten Merkmalswerts durch Ausführen von Bestimmungsprozessen von:

einem ersten Bestimmungsprozess zum Bestimmen eines Dunkelraumbildes auf der Basis von mindestens einem der Mehrzahl von Pixeln kleiner als ein erster vorbestimmter Wert in dem Bild und einem Verhältnis von Pixeln kleiner als der erste vorbestimmte Wert in dem Bild, und einem Merkmalswert auf der Basis von der in dem Merkmalswertberechnungsprozess berechneten Helligkeit,

einem zweiten Bestimmungsprozess zum Bestimmen eines Starklichtbildes auf der Basis von mindestens einem der Anzahl von Pixeln größer als ein zweiter vorbestimmter Wert in dem Bild und einem Verhältnis von Pixeln größer als der zweite vorbestimmter Wert in dem Bild, und einem Merkmalswert auf der Basis der in dem Merkmalswertberechnungsprozess berechneten Helligkeit;

einem dritten Bestimmungsprozess zum Bestimmen eines Fremdsubstanzbildes auf der Basis von mindestens einem der Anzahl von Pixeln mit einem vorbestimmten Ton in dem Bild und einem Verhältnis von Pixeln, die den vorbestimmten Ton in dem Bild aufweisen, und einem Merkmalswert auf der Basis des in dem Merkmalswertberechnungsprozess berechneten Tons,

einem vierten Bestimmungsprozess zum Bestimmen eines exzessiven Nahaufnahmebildes auf der Basis einer linearen Diskriminanzfunktion unter Verwendung des Merkmalwerts auf der Basis des Tons, und einem fünften Bestimmungsprozess zum Bestimmen eines defokusierten Bildes durch Vergleichen des Merkmalwerts auf der Basis der Frequenz mit einem vorbestimmten Schwellwert; wobei

der Bildaufnahmezustandsbestimmungsprozess ausführt:

Klassifizieren des für die Beobachtung unbrauchbaren Bildes in dem Bildaufnahmezustand als das Dunkelraumbild basierend auf dem Bestimmungsergebnis des ersten Bestimmungsprozesses,

Klassifizieren des für die Beobachtung unbrauchbaren Bildes in dem Bildaufnahmezustand als das Starklichtbild basierend auf dem Bestimmungsergebnis des zweiten Bestimmungsprozesses,

Klassifizieren des für die Beobachtung unbrauchbaren Bildes in dem Bildaufnahmezustand als das Fremdsubstanzbild basierend auf dem Bestimmungsergebnis des dritten Bestimmungsprozesses,

Klassifizieren des für die Beobachtung unbrauchbaren Bildes in dem Bildaufnahmezustand als das exzessive Nahaufnahmebild basierend auf dem Bestimmungsergebnis des vierten Bestimmungsprozesses und

Klassifizieren des für die Beobachtung unbrauchbaren Bilden in dem Bildaufnahmeprozess als das defokusierte Bild basierend auf dem Bestimmungsergebnis des fünften Bestimmungsprozesses; und

einen Festlegungsprozess zum Festlegen eines Flags zum Angeben eines für die Beobachtung unbrauchbaren Bildes in dem Bildaufnahmezustand oder eines für die Beobachtung brauchbaren Bildes in einem Bildaufnahmezustand, für jedes der Bilder gemäß einem Bestimmungsergebnis des Bildaufnahmezustandsbestimmungsprozesses.

**2.** Bildverarbeitungsverfahren nach Anspruch 1, bei dem die Frequenzkomponente durch einen Prozess extrahiert wird, der einen Filterprozess (S63) enthält zum Anlegen einer Bandpassfilterung zum Extrahieren einer Frequenzkomponente, die eine lebende Schleimoberflächenstrukturkomponente in dem Bild darstellt, und einem Frequenzleistungsberechnungsprozess (S65) zum Berechnen von Frequenleistung der extrahierten Frequenzkomponente; wobei

in dem Merkmalswertberechnungsprozess ein Berechnungsergebnis des Frequenzleistungsberechnungsprozesses als Merkmalswert festgelegt wird.

**Revendications**

1. Procédé de traitement d'image comprenant :

un processus de calcul de valeur de caractéristique (S2, S4 ; S12, S13 ; 522, 523, S24 ; S52, S53, S54 ; S62, S63, S64, S65) pour calculer une ou plusieurs valeur(s) de caractéristique(s) de chacune parmi une pluralité d'images incluant une pluralité de signaux de couleur obtenus en capturant une image d'un sujet, sur la base d'une composante de luminosité et de tonalité et de fréquence pour chaque pixel constituant l'image ;

un processus de détermination d'état d'image capturée (S4, S5 ; S14, S15 ; 525, S26 ; S55, S56 ; S66, S67) pour déterminer si chacune des images est ou non une image dans un état d'image capturée inapproprié à une observation, à partir d'un état d'image capturée de chacune des images sur la base de la valeur de caractéristique calculée en exécutant des processus de détermination parmi :

un premier processus de détermination pour déterminer une image d'espace aveugle sur la base d'au moins un parmi le nombre de pixels plus petit qu'une première valeur prédéterminée dans l'image et une proportion de pixels plus petite que la première valeur prédéterminée dans l'image, et d'une valeur de caractéristique sur la base de la luminosité calculée dans le processus de calcul de valeur de caractéristique,

un deuxième processus de détermination pour déterminer une image en grande lumière sur la base d'au moins un parmi le nombre de pixels plus grand qu'une deuxième valeur prédéterminée dans l'image et une proportion de pixels plus grande que la deuxième valeur prédéterminée dans l'image, et d'une valeur de caractéristique sur la base de la luminosité calculée dans le processus de calcul de valeur de caractéristique,

un troisième processus de détermination pour déterminer une image de substance étrangère sur la base d'au moins un parmi le nombre de pixels ayant une tonalité prédéterminée dans l'image et une proportion de pixels ayant la tonalité prédéterminée dans l'image, et d'une valeur de caractéristique sur la base de la tonalité calculée dans le processus de calcul de valeur de caractéristique,

un quatrième processus de détermination pour déterminer une image en gros plan excessif sur la base d'une fonction discriminante linéaire en utilisant la valeur de caractéristique sur la base de la tonalité, et

un cinquième processus de détermination pour déterminer une image non focalisée en comparant la valeur de caractéristique sur la base de la fréquence avec une valeur de seuil prédéterminée ;

le processus de détermination d'état d'image capturée exécutant :

un classement de l'image dans l'état d'image capturée inapproprié à une observation comme l'image d'espace aveugle sur la base du résultat de détermination du premier processus de détermination,

un classement de l'image dans l'état d'image capturée inapproprié à une observation comme l'image en grande lumière sur la base du résultat de détermination du deuxième processus de détermination,

un classement de l'image dans l'état d'image capturée inapproprié à une observation comme l'image de substance étrangère sur la base du résultat de détermination du troisième processus de détermination,

un classement de l'image dans l'état d'image capturée inapproprié à une observation comme l'image en gros plan excessif sur la base du résultat de détermination du quatrième processus de détermination, et

un classement de l'image dans l'état d'image capturée inapproprié à une observation comme l'image non focalisée sur la base du résultat de détermination du cinquième processus de détermination ; et

un processus de fixation pour fixer un fanion pour indiquer comme étant une image dans l'état d'image capturée inapproprié à une observation ou une image dans un état d'image capturée approprié à une observation, pour chacune des images en fonction d'un résultat de détermination du processus de détermination d'état d'image capturée.

2. Procédé de traitement d'image selon la revendication 1, dans lequel la composante de fréquence est extraite par des processus incluant un processus de filtrage (S63) pour appliquer un filtrage passe-bande pour extraire une composante de fréquence constituant une composante structurelle de surface de muqueuse vivante dans l'image et un processus de calcul de fréquence-puissance (S65) pour calculer une fréquence-puissance de la composante de fréquence extraite ; et

dans le processus de calcul de valeur de caractéristique, un résultat de calcul par le processus de calcul de fréquence-puissance est fixé comme une valeur de caractéristique.

# FIG.1A

# FIG.1B

# FIG.2

# FIG.3

| | | | | | |
|---|---|---|---|---|---|
| 11a | 11b | 11c | 11d | | 4 |

ANTENNA SWITCH — 45

ANTENNA SELECTION — 46

3

23

TRANSMITTER — 37

RECEIVER — 33

IMAGE CAPTURING DEVICE — 43

5

SIGNAL PROCESSING — 35

LIQUID CRYSTAL MONITOR — 12

MEMORY — 47

# FIG.4

1 FRAME PERIOD

| INTENSITY RECEIVING PERIOD | IMAGE SIGNAL PERIOD |
|---|---|

# FIG.5

# FIG.6

# FIG.7

# FIG.8

# FIG.9

# FIG.10

```
        ┌─────────────────────┐
        │        START        │
        │  (j-TH IMAGE INPUT)  │
        └─────────────────────┘
                   │
                   ▼
        ┌─────────────────────┐
        │       i = 1         │
        │      Cnt = 0        │───── S1
        │    D[j] = FALSE     │
        └─────────────────────┘
                   │
                   ▼
              S2 ◇
         (ri ≦ Td) & (gi ≦ Td)      NO
           & (bi ≦ Td)?
                   │ YES
                   ▼
        ┌─────────────────────┐
        │     Cnt = Cnt+1     │───── S3
        └─────────────────────┘
                   │
                   ▼
        YES     S4 ◇
    ┌──────────  Cnt ≧ α?
    │              │ NO
    ▼
  S5                ▼
┌──────────┐    S6 ◇
│ D[j] =   │   i = ISX × ISY?        NO
│  TRUE    │       │ YES
└──────────┘       ▼                  S7
        ┌─────────────────────┐   ┌─────────┐
        │        END          │   │ i = i+1 │
        │ (TO (j+1)-TH IMAGE)  │   └─────────┘
        └─────────────────────┘
```

27

# FIG.11

```
                    ┌─────────────────────────┐
                    │         START           │
                    │   (j-TH IMAGE INPUT)     │
                    └─────────────────────────┘
                                 │
                                 ▼
                    ┌─────────────────────────┐
                    │         i = 1           │
                    │        Cnt = 0          │────S11
                    │      H[j] = FALSE        │
                    └─────────────────────────┘
                                 │               S12
                                 ▼
                          ╱─────────────╲        NO
                        ╱  (ri ≧ Th) & (gi ≧ Th) ╲──────────┐
                        ╲  & (bi ≧ Th)?         ╱            │
                          ╲─────────────╱                   │
                                 │ YES                       │
                                 ▼                           │
                    ┌─────────────────────────┐              │
                    │       Cnt = Cnt+1        │────S13       │
                    └─────────────────────────┘              │
                                 │           S14             │
                                 ▼                           │
         S15          YES  ╱───────────╲                     │
          │         ┌─────╱  Cnt ≧ β?    ╲                   │
          ▼         │     ╲───────────╱                     │
  ┌──────────────┐  │          │ NO                          │
  │  H[j] = TRUE │  │          ▼◄──────────────────────────┘
  └──────────────┘  │     S16
          │         │          ╱───────────╲      NO
          │         │        ╱ i = ISX × ISY? ╲──────────┐
          │         │          ╲───────────╱      S17     │
          │         │               │ YES                  ▼
          │         │               ▼             ┌──────────────┐
          │         └──────────►┌─────────────┐   │    i = i+1   │
          │                     │     END     │   └──────────────┘
          └─────────────────────│(TO (j+1)-TH │
                                │   IMAGE)    │
                                └─────────────┘
```

# FIG.12

START
(j-TH IMAGE INPUT)

i = 1
Cnt = 0
A1[j] = FALSE — S21

x = atan(gi/ri)
y = atan(bi/ri) — S22

S23
IS (x, y)
DISTRIBUTED IN
FOREIGN SUBSTANCE
TONE AREA?

NO

YES

Cnt = Cnt+1 — S24

S25
Cnt ≧ γ?

YES
S26
A1[j] = TRUE

NO

S27
i = ISX × ISY?

NO
S28
i = i+1

YES

END
(TO (j+1)-TH IMAGE)

# FIG.13

| V1 | V2 | atan (V1/V2) |
|:---:|:---:|:---:|
| 0 | 0 | 0 |
| 0 | 1 | 0 |
| 0 | 2 | 0 |
| 0 | 3 | 0 |
| ⋮ | ⋮ | ⋮ |
| ⋮ | ⋮ | ⋮ |
| 255 | 252 | 45.339 |
| 255 | 253 | 45.225 |
| 255 | 254 | 45.112 |
| 255 | 255 | 45 |

# FIG.14

AREA (2) [LIVING MUCOUS TONE DISTRIBUTION AREA]

AREA (1) [FOREIGN SUBSTANCE TONE DISTRIBUTION AREA]

30

# FIG.15

```
           START
      (j-TH IMAGE INPUT)
              │
              ▼
         i = 1
         CntD = 0
         CntH = 0          ─── S31
         CntA = 0
         N[j] = FALSE
              │
              ▼ ◄──────────────────────────────────────┐
                            S32                          │
         (ri ≦ Td) & (gi ≦ Td)  ──── NO ──┐              │
           & (bi ≦ Td)?                    │             │
              │ YES                        │             │
              ▼                            │             │
         CntD = CntD+1  ─── S33            │             │
              │                            │             │
              ▼           S34              │             │
    YES ── CntD ≧ α?                       │             │
     │        │ NO ◄───────────────────────┘             │
     │        ▼                                          │
     │                    S35                            │
     │   (ri ≧ Th) & (gi ≧ Th)  ──── NO ──┐              │
     │     & (bi ≧ Th)?                    │             │
     │        │ YES                        │             │
     │        ▼                            │             │
     │   CntH = CntH+1  ─── S36            │             │
     │        │                            │             │
     │        ▼           S37              │             │
     ├── YES  CntH ≧ β?                    │             │
     │        │ NO ◄───────────────────────┘             │
     │        ▼                                          │
     │   x = atan(gi/ri)  ─── S38                        │
     │   y = atan(bi/ri)                                 │
     │        │                                          │
     │        ▼           S39                            │
     │      IS (x, y)                                    │
     │   DISTRIBUTED IN  ──── NO ──┐                     │
     │   FOREIGN SUBSTANCE          │                    │
     │     TONE AREA?               │                    │
     │        │ YES                 │                    │
     │        ▼                     │                    │
     │   CntA = CntA+1  ─── S40     │                    │
     │        │                     │                    │
     │        ▼           S41       │                    │
     ├── YES  CntA ≧ γ?             │                    │
     │        │ NO ◄────────────────┘                    │
     ▼        ▼             S43                  S44      │
  S42    i = ISX × ISY?  ──── NO ──►  i = i+1 ──────────┘
         │
 N[j] = TRUE  │ YES
     │        ▼
     └──►  END
      (TO (j+1)-TH IMAGE)
```

# FIG.16

```
┌─────────────────────────────┐
│           START             │
│     (j-TH IMAGE INPUT)       │
└─────────────────────────────┘
              │
              ▼
┌─────────────────────────────┐
│       A2[j] = FALSE          │ ──S51
└─────────────────────────────┘
              │
              ▼
┌─────────────────────────────┐
│         DARK SPACE,          │
│ HIGH LIGHT PIXELS EXCLUDED   │ ──S52
└─────────────────────────────┘
              │
              ▼
┌─────────────────────────────┐
│  CALCULATION OF mean(gi/ri), │
│ mean(bi/ri), σ(gi/ri), σ(bi/ri) │ ──S53
└─────────────────────────────┘
              │
              ▼
┌─────────────────────────────┐
│    APPLICATION OF LINEAR     │
│    DISCRIMINANT FUNCTION     │ ──S54
└─────────────────────────────┘
              │
              ▼              S55
          ◇─────────────────────◇
    YES  ╱   EXCESSIVELY         ╲
  ◀──────   CLOSE-UP IMAGE?       
S56     ╲                       ╱
         ◇─────────────────────◇
┌──────────────┐         │ NO
│ A2[j] = TRUE │         │
└──────────────┘         │
      │                  │
      └──────────────────┤
                         ▼
            ┌─────────────────────────────┐
            │            END               │
            │    (TO (j+1)-TH IMAGE)        │
            └─────────────────────────────┘
```

# FIG.17

```
┌─────────────────────────────┐
│           START             │
│     (j-TH IMAGE INPUT)       │
└─────────────────────────────┘
              │
              ▼
┌─────────────────────────────┐
│        A3[j] = FALSE         │──S61
└─────────────────────────────┘
              │
              ▼
┌─────────────────────────────┐
│   DARK SPACE, HIGH LIGHT     │
│  PIXELS DETERMINATION STORED │──S62
└─────────────────────────────┘
              │
              ▼
┌─────────────────────────────┐
│      BAND PASS FILTERING     │──S63
└─────────────────────────────┘
              │
              ▼
┌──────────────────────────────────────┐
│  EXTRACTION COMPONENT BORDERING       │
│ DARK SPACE/HIGH LIGHT PIXEL EXCLUDED  │──S64
│  EXCLUDED NUMBER OF PIXELS COUNT      │
└──────────────────────────────────────┘
              │
              ▼
┌─────────────────────────────┐
│   CALCULATION OF STRUCTURAL  │──S65
│  COMPONENT FEATURE VALUE μ    │
└─────────────────────────────┘
              │
              ▼       S66
          ◇─────────────◇
   YES   /   μ ≦ Tf?     \
  ◄──────            
S67       ◇─────────────◇
   │            │ NO
   ▼            │
┌──────────┐    │
│A3[j]=TRUE│    │
└──────────┘    │
   │            │
   └────────────┤
                ▼
┌─────────────────────────────┐
│            END              │
│     (TO (j+1)-TH IMAGE)      │
└─────────────────────────────┘
```

# FIG.18

**FIG.19A**

**FIG.19B**

**FIG.19C**

**FIG.19D**

# FIG.20

```
                    ┌─────────────────────┐
                    │       START         │
                    │ (j-TH IMAGE INPUT)  │
                    └─────────────────────┘
                              │
                              ▼
                    ┌─────────────────────┐
                    │      C[j] = 0        │──── S71
                    └─────────────────────┘
                              │
                              ▼  S72
                         ◇ DARK SPACE ◇        YES
                         ◇  IMAGE?   ◇ ──────────────────────► S73
                              │ NO                          ┌──────────┐
                              ▼        S74                  │ C[j] = 1 │
                      ◇ HIGH LIGHT ◇      YES               └──────────┘
                      ◇   IMAGE?   ◇ ────────────┐
                              │ NO          S75  ▼
                              ▼   S76        ┌──────────┐
          YES          ◇  FOREIGN  ◇         │ C[j] = 2 │
    S77 ◄──────────────◇ SUBSTANCE ◇         └──────────┘
   ┌──────────┐        ◇  IMAGE?   ◇
   │ C[j] = 3 │              │ NO
   └──────────┘              ▼   S78
              YES     ◇ EXCESSIVELY ◇
        S79 ◄─────────◇ CLOSE-UP IMAGE? ◇
       ┌──────────┐         │ NO
       │ C[j] = 4 │         ▼   S80
       └──────────┘    ◇   OTHER    ◇   YES
                       ◇ OBSERVATION ◇ ──────► S81
                       ◇INAPPROPRIATE◇    ┌──────────┐
                       ◇  IMAGES?   ◇     │ C[j] = 5 │
                            │ NO          └──────────┘
                            ▼
                    ┌─────────────────────┐
                    │        END          │
                    │ (TO (j+1)-TH IMAGE) │
                    └─────────────────────┘
```

# FIG.21

```
          ┌──────────────────┐
          │      START        │
          └──────────────────┘
                   │
                   ▼
          ┌──────────────────┐
          │       j = 1       │ ──S91
          └──────────────────┘
                   │
                   ▼
              ◇ S92
        ╱─────────────────╲
       ╱                   ╲      NO
      ◇     C[j] = 0?       ◇ ──────────┐
       ╲                   ╱            │
        ╲─────────────────╱             │
                │ YES                    │
                ▼                        │
          ┌──────────────────┐          │
          │ j-TH IMAGE DISPLAY│ ──S93    │
          └──────────────────┘          │
                   │◄───────────────────┘
                   ▼
          ┌──────────────────┐
          │     j = j+1       │ ──S94
          └──────────────────┘
                   │
                   ▼   S95
        ╱─────────────────╲
  NO   ╱    ALL IMAGES      ╲
 ◄────◇     FINISHED?        ◇
       ╲                   ╱
        ╲─────────────────╱
                │ YES
                ▼
          ┌──────────────────┐
          │       END         │
          └──────────────────┘
```

# FIG.22

```
        ┌─────────────────────────┐
        │          START          │
        └─────────────────────────┘
                     │
                     ▼
        ┌─────────────────────────┐
        │          j = 1          │──── S101
        └─────────────────────────┘
                     │
          ┌──────────▼──────────┐
          │                     │
          │                   S102
                 ◇─────────────◇
                /               \
               <    C[j] = 0?    >──── NO ──┐
                \               /           │
                 ◇─────────────◇            │
                     │                      │
                    YES                     │
                     ▼                      │
        ┌─────────────────────────┐        │
        │   j-TH IMAGE STORED     │──── S103 │
        └─────────────────────────┘        │
                     │◄─────────────────────┘
                     ▼
        ┌─────────────────────────┐
        │         j = j+1         │──── S104
        └─────────────────────────┘
                     │
                   S105
                 ◇─────────────◇
                /               \
        NO ────<   ALL IMAGES    >
                \   FINISHED?    /
                 ◇─────────────◇
                     │
                    YES
                     ▼
        ┌─────────────────────────┐
        │          END            │
        └─────────────────────────┘
```

**EP 1 806 091 B1**

**REFERENCES CITED IN THE DESCRIPTION**

**Patent documents cited in the description**

- JP 2000023980 A
- EP 1437083 A1
- US 5469840 A
- JP 2003284682 A